# EUROPEAN PATENT SPECIFICATION

(11) **EP 3 599 282 B1**
(45) Date of publication and mention of the grant of the patent: **17.03.2021**
(21) Application number: 19186488.3
(22) Date of filing: 16.07.2019
(51) Int. Cl.: C12P 13/08

(54) **METHOD FOR THE FERMENTATIVE PRODUCTION OF L-LYSINE**
VERFAHREN ZUR FERMENTATIVEN HERSTELLUNG VON L-LYSIN
PROCÉDÉ DE PRODUCTION DE L-LYSINE PAR FERMENTATION

(30) Priority: 24.07.2018 EP 18185087
(43) Date of publication of application: 29.01.2020
(73) Proprietor: Evonik Operations GmbH, 45128 Essen (DE)
(72) Inventor: Schneider, Frank, 33790 Halle (DE); Thierbach, Georg, 33613 Bielefeld (DE); Voß, Kornelia, 33803 Steinhagen (DE); Bekel, Thomas, 33790 Halle (Westf.) (DE)
(74) Representative: Evonik Patent Association

(56) References cited:
- EP-A1- 3 318 637
- Y. TSUGE ET AL: "Deletion of cgR_1596 and cgR_2070, Encoding NlpC/P60 Proteins, Causes a Defect in Cell Separation in Corynebacterium glutamicum R", JOURNAL OF BACTERIOLOGY, vol. 190, no. 24, 15 December 2008 (2008-12-15), pages 8204-8214, XP055521383, US ISSN: 0021-9193, DOI: 10.1128/JB.00752-08

## Description

L-lysine is used in human medicine, in the pharmaceutical industry, in the food industry and particularly in nutrition of animals.

L-lysine is produced by fermentation of strains of the species *Corynebacterium glutamicum.* Because of the great economic importance, work is continually being done on improving the production methods. Improvements may relate to the fermentation technology such as e.g. stirring and supplying oxygen, or to the composition of the nutrient media e.g. the sugar concentration during fermentation, or to the processing of the fermentation broth to a suitable product form by e.g. drying and granulating the fermentation broth or ion exchange chromatography or may relate to the intrinsic performance properties of the microorganism itself.

The methods used for improving the performance properties of these microorganisms are those of mutagenesis, selection and screening of mutants. The strains obtained in this way are resistant to anti-metabolites or are auxotrophic for metabolites of regulatory importance, and produce L-lysine. A well-known anti-metabolite is the L-lysine analogue S-(2-aminoethyl)-L-cysteine (see e.g. Tosaka et al.: Agricultural and Biological Chemistry 42(4), 745-752, (1978)).
Methods of recombinant DNA technology have likewise been used for a number of years for improvement of L-lysine-producing strains of the species *Corynebacterium glutamicum,* by modifying, i.e. enhancing or attenuating, individual genes involved in L-lysine biosynthesis and investigating the effect on L-lysine production.

Research on the control of cell lysis of *Corynebacterium glutamicum,* which may cause decreased productivity during fermentation, is still insufficient. Cell wall hydrolases are known as enzymes that degrade bacterial cell walls, and are present in all microorganisms having peptidoglycan containing cell walls (Rice KC & Bayles KW, Microbiology and Molecular Biology Reviews 2008, 72:85-109). Although research on such cell wall hydrolases has been conducted in various bacteria, their precise control mechanism is still unknown.

The nucleotide sequences of the chromosomes of various bacteria or strains resp. of the species *Corynebacterium glutamicum,* and their analysis have been disclosed. This information is available at publicly accessible databases and may be used for strain development purposes. One such database is the GenBank data base of the NCBI (National Center for Biotechnology Information, U.S. National Library of Medicine 8600 Rockville Pike, Bethesda MD, 20894 USA).

During the annotation procedure for a sequenced chromosome of an organism identified structures such as e.g. genes or coding sequences are furnished with a unique identifier called locus_tag by the supplier of the information to the data base.

The nucleotide sequence of the *Corynebacterium glutamicum* ATCC13032 chromosome and its analysis were described by Ikeda and Nakagawa (Applied Microbiology and Biotechnology 62, 99-109 (2003)) and in EP1108790. The information is available at the NCBI under accession number NC_003450. In the chromosome sequence disclosed under accession number NC_003450 locus_tag NCgl1480 identifies a nucleotide sequence starting with a ttg codon coding for a cell wall-associated hydrolase. The amino acid sequence of the encoded polypeptide having a length of 604 amino acids is available under the identifier NP_600753. In EP1108790 A2 the coding sequence is disclosed under sequence 7064 (see GenBank accession number AX127148).

The nucleotide sequence of the *Corynebacterium glutamicum* ATCC13032 chromosome and its analysis were independently described by Kalinowski et al. (Journal of Biotechnology 104 (1-3), 5-25 (2003)). The information is available at the NCBI under accession number NC_006958. Locus_tag CGTRNA_RS07715 identifies a nucleotide sequence coding for a hydrolase and starting with a ttg codon. The old_locus_tag designation cg1735 is also used in the art. The amino acid sequence of the polypeptide having a length of 604 amino acids is available under the identifier WP_011014435.

The nucleotide sequences of locus_tag NCgl1480 and CGTRNA_RS07715 are identical.

Marchard et al. (Journal of Bacteriology 194(3), 587-597 (2012) describe NCgl1480 as a member of a group of putative mycomembrane proteins more specifically as a putative cell wall associated hydrolase bearing a signal sequence for the general secretory pathway for cell membrane translocation.

EP3318637 A1 describes the inactivation of various cell wall hydrolases and its effect on L-lysine production. The document further discloses that the inactivation of the cell wall hydrolase encoded by the nucleotide sequence of NCgl1480 by deleting said nucleotide sequence enhanced the production of L-lysine. EP3318637 A1 also suggests a method of replacing the gene encoding the enzyme on the chromosome with a mutated gene so that the enzyme activity can be reduced. However the document does not teach such mutated gene. Accordingly, there is a need to provide such mutated variant(s).

Information concerning transcription signals in *Corynebacterium glutamicum,* e.g. -10 region of a promoter, or transcriptional start site (TSS) of the gene identified by old_locus_tag cg1735 can be found in Pfeifer-Sancar et al. (BMC Genomics 14:888 (2013)), Albersmeier et al. (Journal of Biotechnology 257 (2017) 99-109) or Menz et al. (BMC Genomics 2013, 14:714).

The nucleotide sequence of the *Corynebacterium glutamicum* R chromosome and its analysis was described by Yukawa et al. (Microbiology 153(4):1042-1058 (2007)). It is available at the NCBI under accession number AP009044. Locus_tag cgR_1596 identifies a nucleotide sequence coding for a hypothetical protein and starting with a gtg codon. The amino acid sequence of the polypeptide having a length of 610 amino acids is available under the identifier BAF54588.

The nucleotide sequence of the *Corynebacterium glutamicum* R chromosome and its analysis is also available under accession number NC_009342. Under designation cgR_1596 the predicted function of the polypeptide is given as a hydrolase.

The polypeptide identified by locus_tag cgR_1596 of accession number AP009044 is the homologous counterpart of the polypeptide identified by NCgl1480. Amino acid sequence alignment reveals that besides other differences the polypeptide identified by locus_tag cgR_1596 displays 26 additional amino acids at the N-terminus when compared to the polypeptide identified by NCgl1480 (see Table 1).

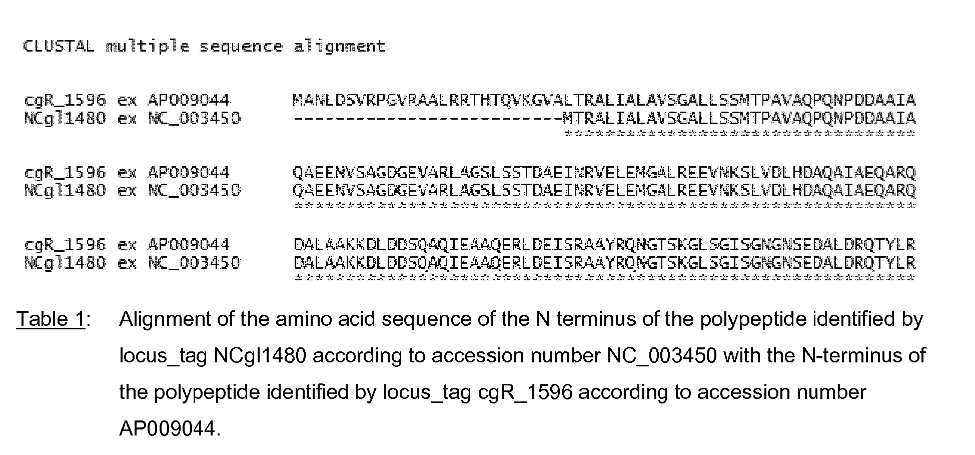

Tsuge et al. (Journal of Bacteriology 190 (24), 8204-8214, 2008) investigated the effect of deletion of cgR_1596 on cell separation in *Corynebacterium glutamicum* R. Tsuge et al. further stated that CgR_1596 contains an amino acid sequence domain conserved in cell wall hydrolases and a signal peptide of 49 amino acids.

Object of the present invention is to provide new measures for the fermentative production of L-lysine by bacteria of the species *Corynebacterium glutamicum.*

To achieve the object outlined above the present invention makes available a novel method for the fermentative production of L-lysine using bacteria of the species *Corynebacterium glutamicum,* having the ability to excrete L-lysine, containing in their chromosome a variant of a polynucleotide encoding the polypeptide shown in SEQ ID NO:2, wherein in the amino acid sequence of the polypeptide, the amino acid at position 90 is isoleucine, the amino acid at position 159 is arginine, the amino acid at position 449 is aspartic acid and the amino acid at position 454 is phenylalanine. The amino acid sequence of said polypeptide variant is also shown under SEQ ID NO:4 of the sequence listing.

The present invention provides a method for the fermentative production of L-lysine comprising the steps of
a) providing a bacterium of the species *Corynebacterium glutamicum,* having the ability to excrete L-lysine, containing in its chromosome a polynucleotide encoding a polypeptide comprising the amino acid sequence of SEQ ID NO:4,
b) cultivating the bacterium in a suitable medium under suitable conditions, and
c) accumulating the L-lysine in the medium to form an L-lysine containing fermentation broth.

It was found that the modified bacteria provided in the method according to the invention excreted L-lysine, into a suitable medium under suitable fermentation conditions in an increased manner with respect to one or more parameters selected from product concentration (e.g. amount of L-lysine produced per volume) as compared to the unmodified bacterium.

It is obvious that a higher product concentration facilitates product manufacturing e.g. purification and isolation. An increased product yield reduces the amount of raw material required. An increased product formation rate reduces the time required for a fermentation run thus increasing the availability of a given fermenter.

The method according to the invention thus contributes to the improvement of technical and economic aspects of the manufacturing of L-lysine or L-lysine containing products.

SEQ ID NO:2 of the sequence listing shows the amino acid sequence of a polypeptide encoded by *Corynebacterium glutamicum* ATCC13032. The difference between the amino acid sequence shown in SEQ ID NO:2 and the amino acid sequence shown under NCgl1480 is a sequence of 26 additional amino acids at the N-terminus of SEQ ID NO:2. The amino acid sequence of SEQ ID NO:2 positions 27 to 630 is identical to the amino acid presented under NCgl1480 with the exception of the interpretation of the ttg start codon in NCgl1480 as a methionine codon. SEQ ID NO:4 of the sequence listing shows the amino acid sequence of a variant of SEQ ID NO:2 used in the method according to the invention. The difference between the two amino acid sequences is the following: The amino acid at position 90 of SEQ ID NO:2 is valine whereas in SEQ ID NO:4 it is isoleucine. The amino acid at position 159 of SEQ ID NO:2 is glycine whereas in SEQ ID NO:4 it is arginine. The amino acid at position 449 of SEQ ID NO:2 is glycine whereas in SEQ ID NO:4 it is aspartic acid. The amino acid at position 454 of SEQ ID NO:2 is serine whereas in SEQ ID NO:4 it is phenylalanine.

Accordingly, using the one letter abbreviation code for amino acids, the variant is characterized by the following amino acid substitutions or amino acid exchanges resp.: V90I, G159R, G449D and S454F with SEQ ID NO:2 as reference.

Using the information given under NCgl1480, the positions for said amino acid substitutions or amino acid exchanges resp. would be assigned as: V64I, G133R, G423D and S428F.

In a preferred embodiment the bacterium provided in the method according to the invention contains in its chromosome a polynucleotide encoding an amino acid sequence of a polypeptide shown in SEQ ID NO:4 comprising the nucleotide sequence of positions 923 to 2812 of SEQ ID NO:3.

Further in a preferred embodiment the bacterium provided in the method according to the invention contains in its chromosome a polynucleotide encoding an amino acid sequence of a polypeptide shown in SEQ ID NO:4 comprising the nucleotide sequence of positions 923 to 2815 of SEQ ID NO:3.

Following the descriptions in the art the polypeptide shown in SEQ ID NO:4 or a processed derivative thereof, e.g. after cleaving off the signal peptide, is a mycomembrane protein and has the function or activity resp. of a cell wall-associated hydrolase, cell wall hydrolase or hydrolase.

In a further embodiment the bacterium provided in the method according to the invention comprises, in addition to the polynucleotide encoding the polypeptide shown in SEQ ID NO:4, a polynucleotide encoding a polypeptide identified by NCgl2986, wherein the amino acid proline at position 265 of the amino acid sequence is substituted by a different amino acid, preferably by Leucine.

The amino acid sequence of the polypeptide identified by NCgl2986 is also shown under SEQ ID NO:8 of the sequence listing. The nucleotide sequence encoding said polypeptide is shown under SEQ ID NO:7 of the sequence listing.

In the art the function of polypeptide identified by NCgl2986 is described as a N-acetylmuramoyl-L-alanine amidase.

The term L-lysine, where mentioned herein, in particular in the context of product formation, also comprises their ionic forms and salts, for example L-lysine mono hydrochloride or L-lysine sulfate.

For practicing the present invention bacteria of the species *Corynebacterium glutamicum* are used. A description of the genus *Corynebacterium* and the species *Corynebacterium glutamicum* comprised by this genus can be found in the article *"Corynebacterium"* by K. A. Bernard and G. Funke in Bergey's Manual of Systematics of Archaea and Bacteria (Bergey's Manual Trust, 2012).

Suitable bacteria for the method of this invention are L-lysine excreting strains of *Corynebacterium glutamicum,* for example L-lysine excreting strains obtained by one or several steps of strain development from strain ATCC13032 and the like and modified as described in this invention.

Strain ATCC13032 (also available as DSM20300) is the taxonomic type strain of the species *Corynebacterium glutamicum.* A taxonomic study of this group of bacteria based on DNA-DNA hybridization was done by Liebl et al. (International Journal of Systematic Bacteriology 41(2), 255-260, 1991). A comparative analysis of various strains of the species *Corynebacterium glutamicum* based on genome sequence analysis was provided by Yang and Yang (BMC Genomics 18(1):940, 2017).

A multitude of L-lysine excreting strains of the genus *Corynebacterium,* in particular of the species *Corynebacterium glutamicum* were obtained in the art during the past decades starting from strains such as ATCC13032 and the like. They were obtained as a result of strain development programs using inter alia methods like classical mutagenesis, selection for antimetabolite resistance as well as amplification and promotor modification of genes of the biosynthetic pathway of the L-lysine by genetic engineering methods. Summaries may be found in L. Eggeling and M. Bott (Handbook of Corynebacterium glutamicum, CRC Press, 2005) or H. Yukawa and M. Inui (Corynebacterium glutamicum Biology and Biotechnology, Springer Verlag, 2013) or A. Yokota and M. Ikeda (Amino Acid Fermentation, Springer Verlag, 2017).

L-lysine excreting strains of the species *Corynebacterium glutamicum* are widely known in the art and can be modified as described in the present invention. For example Blombach et al. (Applied and Environmental Microbiology 75(2), 419-427, 2009) describe strain DM1933, which is deposited under accession DSM25442 according to the Budapest treaty. Strain DM1933 was obtained from ATCC13032 by several steps of strain development. Furthermore L-lysine excreting *Corynebacterium glutamicum* strain DM2031, deposited according to the Budapest Treaty as DSM32514 may be used. Strain DM2031 is a further developed derivative of DM1933 having enhanced L-lysine excretion ability. Other L-lysine excreting *Corynebacterium glutamicum* strains are e.g. described in WO2008033001 A1 and EP0841395 A1.

L-lysine excreting strains of the species *Corynebacterium glutamicum* typically contain a polynucleotide coding for a feedback resistant aspartokinase polypeptide variant. A feedback resistant aspartokinase polypeptide variant means an aspartokinase which is less sensitive, or desensitized resp., to inhibition by mixtures of L-lysine and L-threonine, e.g. 10 mM each, or mixtures of the L-lysine analogue S-(2-aminoethyl)-L-cysteine and L-threonine, e.g. 50 mM S-(2-aminoethyl)-L-cysteine and 10 mM L-threonine, when compared to the wild form of the enzyme, which is contained in wild strains like for example ATCC13032, ATCC14067 and ATCC13869. The EC number for aspartokinase is EC 2.7.2.4. Descriptions of polynucleotides of *Corynebacterium glutamicum* encoding a feedback resistant aspartokinase polypeptide variant are for example given in US5688671, US6844176 and US6893848. A summarizing list can be found inter alia in WO2009141330. The symbol used in the art for a gene coding for an aspartokinase polypeptide is *lysC.* In case the gene codes for a feedback resistant polypeptide variant the art typically uses symbols like *lysC^{fbr}* with fbr indicating feedback resistance.

Accordingly, said L-lysine excreting strains of the species *Corynebacterium glutamicum* modified as described in the present invention preferably contain at least (≥) one copy of a polynucleotide coding for a feedback resistant aspartokinase polypeptide.

SEQ ID NO:5 shows the nucleotide sequence of the coding sequence of the aspartokinase polypeptide of strain ATCC13032 and SEQ ID NO:6 the amino acid sequence of the encoded polypeptide. It is known in the art (US6893848) that exchange of the amino acid Thr at position 311 of SEQ ID NO:6 for lie imparts the enzyme feedback resistance to inhibition by mixtures of L-lysine and L-threonine.

Accordingly, it is preferred that the amino acid sequence of said feedback resistant aspartokinase polypeptide comprises the amino acid sequence of SEQ ID NO:6 containing isoleucine at position 311.

Said amino exchange can be achieved by exchanging the nucleobase cytosine (c) at position 932 of SEQ ID NO:5 to give thymine (t) (c→t substitution at the second position of the codon). The acc codon for threonine is thus altered to the atc codon for isoleucine.

It is further known in the art that exchange of the gtg start codon of the coding sequence for the aspartokinase polypeptide for atg enhances expression of the polypeptide (e.g. EP2796555 A2). Accordingly, it is preferred that the sequence coding for a feedback resistant aspartokinase polypeptide begins with an atg start codon.

Summaries concerning the breeding of L-lysine excreting strains of *Corynebacterium glutamicum* may be found inter alia in L. Eggeling and M. Bott (Handbook of Corynebacterium glutamicum, CRC Press, 2005), V. F. Wendisch (Amino Acid Biosynthesis - Pathways, Regulation and Metabolic Engineering, Springer Verlag, 2007), H. Yukawa and M. Inui (Corynebacterium glutamicum Biology and Biotechnolgy, Springer Verlag, 2013), and Eggeling and Bott (Applied Microbiology and Biotechnology 99 (9), 3387-3394, 2015).

The term DSM denotes the depository Deutsche Sammlung für Mikroorganismen und Zellkulturen located in Braunschweig, Germany. The term ATCC denotes the depository American Type Culure Collection located in Manasass, Virginia, US.

Details regarding the biochemistry and chemical structure of polynucleotides and polypeptides as present in living things such as bacteria like *Corynebacterium glutamicum* or *Escherichia coli,* for example, can be found inter alia in the text book "Biochemie" by Berg et al. (Spektrum Akademischer Verlag Heidelberg, Berlin, Germany, 2003; ISBN 3-8274-1303-6).

For sequence analysis of polynucleotides and polypeptides, e.g. sequence alignments the Clustal W program (Larkin et al.: Clustal W and Clustal X version 2.0. In: Bioinformatics 23, 2947-2948 (2007)) or public or commercially available software such as the CLC Genomics Workbench (Qiagen, Hilden, Germany) or the program MUSCLE provided by the European Bioinformatics Institute (EMBL-EBI, Hinxton, UK) may be used. For translation of the genetic code codon table 11 provided by the NCBI was used.

*Corynebacterium glutamicum,* in particular strain ATCC13032 and L-lysine excreting strains obtained therefrom during a strain development program, contain in their chromosome a gene, in particular one gene, encoding a polypeptide having the activity of a cell wall associated hydrolase, also referred to as cell wall hydrolase herewith, and comprising the amino acid sequence of SEQ ID NO:2. The coding sequence is shown in SEQ ID NO:1 positions 923 to 2812. The coding sequence may contain silent mutations which do not alter the amino acid sequence of the polypeptide. This context is also known as degeneracy of the genetic code in the art. Information concerning the promoter of the gene as published by Pfeifer-Sancar et al. under the old_locus_tag cg_1735 is shown in SEQ ID NO:1 (and SEQ ID NO:3).

During the work for the present invention it was found that modifying L-lysine excreting bacteria of the species *Corynebacterium glutamicum* by exchanging, in the encoded amino acid sequence of the polypeptide shown in SEQ ID NO:2, the amino acid valine at position 90 to isoleucine, the amino acid glycine at position 159 to arginine, the amino acid glycine at position 449 to aspartic acid and the amino acid serine at position 454 to phenylalanine increased their ability to excrete L-lysine in a fermentative process as compared to the unmodified bacterium.

During the work for the present invention it was found that additionally modifying said bacteria containing said amino acid substitutions at positions 90, 159, 449 and 454 of SEQ ID NO:2 by substituting the amino acid proline at position 265 of of the amino acid sequence of the polypeptide shown in SEQ ID NO:8 by a different amino acid, preferably leucine, further increased the ability of said bacterium to excrete I-lysine.

Further it is possible to substitute the amino acid serine at position 590 of SEQ ID NO:2 with phenylalanine in addition to the aforementioned amino acid substitutions. Still further it is obvious to try other amino acid substitutions at the indicated positions of SEQ ID NO:2 and to study the effect on L-lysine excretion of an appropriate *Corynebacterium glutamicum* strain.

During the work for the present invention SEQ ID NO:2 and SEQ ID NO:4 were analyzed for presence and location of signal peptide cleavage sites using SignalP software (DTU Bioinformatics, Department of Bio and Health Informatics, Lyngby, Denmark). As a result of this study the amino acid sequence between positions 49 and 50 was identified as a cleavage site and accordingly the amino acid sequence from position 1 to 49 of SEQ ID NO:2 or SEQ ID NO:4 was identified as a signal peptide for membrane translocation. It is obvious that the positions of the amino acid substitutions in the polypeptide thus processed change accordingly.

The skilled partisan is aware of a number of methods of mutagenesis how to achieve said modification(s) in the *Corynebacterium glutamicum.*

A mutant bacterium according to the invention can be obtained by classical *in vivo* mutagenesis executed with cell populations of strains of *Corynebacterium glutamicum* using mutagenic substances, e.g. N-methyl-N'-nitro-N-nitrosoguanidine, or ultra violet light.

The nucleotide sequence comprising the site(s) of mutagenesis can be amplified by PCR using primers selected from SEQ ID NO:1 or SEQ ID NO:3. By sequencing the PCR product the desired mutants are identified. Details concerning this approach can be found inter alia in US7754446. Real-time PCR in combination with FRET hybridization probes may also be used for mutation detection. The term FRET is the abbreviation for fluorescence resonance energy transfer. Cyril D S Mamotte (The Clinical Biochemist Reviews 27, 63-75 (2006)) reviews the identification of single nucleotide substitutions using this method. Further summaries concerning this method may be found in the textbook Lewin's Genes XII by Jocelyn E. Krebs, Elliott S. Goldstein and Stephan T. Kilpatrick (Jones and Bartlett Publishers, US, 2018) or elsewhere in the art.

Another common method of mutating genes of *Corynebacterium glutamicum* is the method of gene replacement described by Schwarzer and Pühler (Bio/Technology 9, 84-87 (1991)) and further elaborated by Schafer et al. (Gene 145, 69-73 (1994)).

Peters-Wendisch et al. (Microbiology 144, 915-927 (1998)) used the gene replacement method to inactivate the *pyc* gene of *Corynebacterium glutamicum* encoding pyruvate carboxylase. In US7585650 the method was applied to the *zwf* gene to realize an amino acid exchange at position 321 of the amino acid sequence of the Zwf sub-unit of the glucose 6-phosphate dehydrogenase. In US7754446 the method was applied to the *rel* gene to realize an amino acid exchange at position 38 of the amino acid sequence of the GTP- pyrophosphate kinase polypeptide.

In the gene replacement method, a mutation, for example, a deletion, insertion or substitution of at least one nucleobase or of one or more nucleobases resp., is provided by an isolated polynucleotide comprising the nucleotide sequence of the gene in question or a part thereof containing the mutation.

In the context of the present invention the nucleotide sequence of the gene in question is the nucleotide sequence shown under SEQ ID NO:1 positions 923 to 2812 of the sequence listing encoding valine at position 90, glycine at position 159, glycine at position 449 and serine at position 454 of the amino acid sequence (see SEQ ID NO:2).

In the context of the present invention a preferred version of the mutation comprises the following nucleobase substitutions:
- in the gtc codon (shown in positions 1190 to 1192 of SEQ ID NO:1) coding for the amino acid valine at position 90 of SEQ ID NO:2 the nucleobase guanine is substituted for adenine to give the atc codon (shown in positions 1190 to 1192 of SEQ ID NO:3) for isoleucine at position 90 (g →a substitution at position 1190 of SEQ ID NO:1),
- in the ggg codon (shown in positions 1397 to 1399 of SEQ ID NO:1) coding for the amino acid glycine at position 159 of SEQ ID NO:2 the nucleobase guanine at position 1397 of SEQ ID NO:1 is substituted for adenine to give the agg codon (shown in positions 1397 to 1399 of SEQ ID NO:3) for arginine at position 159 (g →a substitution at position 1397 of SEQ ID NO:1),
- in the ggt codon (shown in positions 2267 to 2269 of SEQ ID NO:1) coding for the amino acid glycine at position 449 of SEQ ID NO:2 the nucleobase guanine at position 2268 of SEQ ID NO:1 is substituted for adenine to give the gat codon (shown in positions 2267 to 2269 of SEQ ID NO:3) for aspartic acid at position 449 (g →a substitution at position 2268 of SEQ ID NO:1), and
- in the tcc codon (shown in positions 2282 to 2284 of SEQ ID NO:1) coding for the amino acid serine at position 454 of SEQ ID NO:2 the nucleobase cytosine at position 2283 of SEQ ID NO:1 is substituted for thymine to give the ttc codon (shown in positions 2282 to 2284 of SEQ ID NO:3) for phenylalanine at position 454 (c →t substitution at position 2283 of SEQ ID NO:1).

As a result of the mutation comprising said nucleobase substitutions the altered coding sequence shown in SEQ ID NO:3 positions 923 to 2812 is obtained encoding isoleucine at position 90, arginine at position 159, aspartic acid at position 446 and phenylalanine at position 454 of the amino acid sequence (see also SEQ ID NO:4).

The degeneracy of the genetic code is well known in the art. It is known that
- the codons for isoleucine are ata, att and atc,
- the codons for arginine are cgt, cgc, cga and cgg,
- the codons for aspartic acid are gat and gac, and that
- the codons for phenylalanine are ttt and ttc.
Accordingly, the skilled artisan is aware how to apply other nucleobase substitutions in order to obtain the amino acid exchanges at positions 90, 159, 449 and 454 of SEQ ID NO:2.

In general terms a mutated nucleotide sequence of the gene in question or a part thereof containing the mutation comprises i) a nucleotide sequence at the 5'-end of the site of mutation, which is also referred to as 5'-flanking sequence or upstream sequence in the art, ii) a nucleotide sequence at the 3'-end of the site of mutation, which is also referred to as 3'-flanking sequence or downstream sequence in the art, and iii) the nucleotide sequence of the site of mutation between i) and ii).

Said 5'-flanking sequence and 3'-flanking sequence required for homologous recombination typically have a length of at least 200 bp, at least 400 bp, at least 600 bp or at least 800 bp. The maximum length typically is 1000 bp, 1500 bp or 2000 bp.

In the case of the present invention the mutation comprises nucleobase substitutions in four codons of the coding sequence of SEQ ID NO:1 distributed between positions 1190 to 2283. Accordingly, if flanking sequences having a length of at least 400 bp are desired the mutated nucleotide sequence comprises SEQ ID NO:3 positions 790 to 2683. If flanking sequences having a length of at least 600 bp are desired the mutated nucleotide sequence comprises SEQ ID NO:3 positions 590 to 2883. If flanking sequences having a length of at least 800 bp are desired the mutated nucleotide sequence comprises SEQ ID NO:3 positions 390 to 3083. If flanking sequences having a length of at least 1000 bp are desired the mutated nucleotide sequence comprises SEQ ID NO:3 positions 190 to 3283.

The mutated nucleotide sequence provided is cloned into a plasmid vector, e.g. pK18mobsacB described by Schafer et al. (Gene 145, 69-73 (1994)), that is not capable of autonomous replication in *Corynebacterium glutamicum.* Said plasmid vector comprising said mutated nucleotide sequence is subsequently transferred into the desired strain of *Corynebacterium glutamicum* by transformation using electroporation or conjugation. After two events of homologous recombination comprising a recombination event within the 5'-flanking sequence provided by the plasmid vector with the homologous sequence of the *Corynebacterium glutamicum* chromosome and a recombination event within the 3'-flanking sequence provided by the plasmid vector with the homologous sequence of the *Corynebacterium glutamicum* chromosome, one effecting integration and one effecting excision of said plasmid vector, the mutation is incorporated in the *Corynebacterium glutamicum* chromosome. Thus the nucleotide sequence of the gene in question contained in the chromosome of said desired strain is replaced by the mutated nucleotide sequence. The presence of the mutation in the desired strain is then confirmed e.g. by analysis of the nucleotide sequence or real-time PCR using FRET as described above.

An event of homologous recombination may also be referred to as crossing over.

It is preferred that the L-lysine excreting *Corynebacterium glutamicum* strains provided for the method of the present invention have the ability to excrete ≥ 0,25 g/l, preferably ≥ 0,5 g/l, particularly preferred ≥ 1,0 g/l, very particularly preferred ≥ 2,0 g/l of L-lysine in a suitable medium under suitable conditions.

In a fermentative process according to the invention a *Corynebacterium glutamicum* modified in accordance with the present invention and having the ability to excrete L-lysine is cultivated in a suitable medium under suitable conditions. Due to said ability to excrete said L-lysine the concentration of the L-lysine increases and accumulates in the medium during the fermentative process and the L-lysine is thus produced.

The fermentative process may be a discontinuous process like a batch process or a fed batch process or a continuous process. A summary concerning the general nature of fermentation processes is available in the textbook by H. Chmiel (Bioprozesstechnik, Spektrum Akademischer Verlag, 2011), in the textbook of C. Ratledge and B. Kristiansen (Basic Biotechnology, Cambridge University Press,2006) or in the textbook of V.C. Hass and R. Pörtner (Praxis der Bioprozesstechnik Spektrum Akademischer Verlag, 2011).

A suitable medium used for the production of L-lysine by a fermentative process contains a carbon source, a nitrogen source, a phosphorus source, inorganic ions and other organic compounds as required.

Suitable carbon sources include glucose, fructose, sucrose as well as the corresponding raw materials like starch hydrolysate, molasses or high fructose corn syrup.

As nitrogen source organic nitrogen-containing compounds such as peptones, meat extract, soy bean hydrolysates or urea, or inorganic compounds such as ammonium sulphate, ammonium chloride, ammonium phosphate, ammonium carbonate, ammonium nitrate, ammonium gas or aqueous ammonia can be used.

As phosphorus source, phosphoric acid, potassium dihydrogen phosphate or dipotassium hydrogen phosphate or the corresponding sodium-containing salts can be used.

Inorganic ions like potassium, sodium, magnesium, calcium, iron and further trace elements etc. are supplied as salts of sulfuric acid, phosphoric acid or hydrochloric acid.

Other organic compounds means essential growth factors like vitamins e. g. thiamine or biotin or L-amino acids e.g. L-homoserine.

The media components may be added to the culture in form of a single batch or be fed in during the cultivation in a suitable manner.

During the fermentative process, the pH of the culture can be controlled by employing basic compounds such as sodium hydroxide, potassium hydroxide, ammonia or aqueous ammonia, or acidic compounds such as phosphoric acid or sulphuric acid in a suitable manner. The pH is generally adjusted to a value of from 6.0 to 8.5, preferably 6.5 to 8.0. To control foaming, it is possible to employ antifoam agents such as, for example, fatty acid polyglycol esters. To maintain the stability of plasmids, it is possible to add to the medium suitable selective substances such as, for example, antibiotics. The fermentative process is preferably carried out under aerobic conditions. In order to maintain these conditions, oxygen or oxygen-containing gas mixtures such as, for example air are introduced into the culture. The fermentative process is carried out, where appropriate, at elevated pressure, for example at an elevated pressure of 0.03 to 0.2 MPa. The temperature of the culture is normally from 25 °C to 40 °C, preferably from 30 °C to 37 °C. In a discontinuous process, the cultivation is continued until an amount of the L-lysine sufficient for being recovered has been formed. The cultivation is then completed. This aim is normally achieved within 10 hours to 160 hours. In continuous processes, longer cultivation times are possible.

Examples of suitable media and culture conditions can be found inter alia in L. Eggeling and M. Bott (Handbook of Corynebacterium glutamicum, CRC Press, 2005) and the patent documents US5770409, US5990350, US 5275940, US5763230 and US6025169.

Thus the fermentative process results in a fermentation broth which contains the desired L-lysine.

A product containing the L-lysine is then recovered or manufactured in liquid or solid from the fermentation broth.

A "fermentation broth" means a medium in which a *Corynebacterium glutamicum* described in the invention has been cultivated for a certain time and under certain conditions.

When the fermentative process is completed, the resulting fermentation broth accordingly comprises:
a) the biomass (cell mass) of the *Corynebacterium glutamicum* of the invention, said biomass having been produced due to propagation of the cells of said *Corynebacterium glutamicum,*
b) the desired L-lysine accumulated during the fermentative process,
c) the organic by-products accumulated during the fermentative process, and
d) the components of the medium employed which have not been consumed in the fermentative process.

The organic by-products include compounds which may be formed by the *Corynebacterium glutamicum* of the invention during the fermentative process in addition to production of the L-lysine.

The fermentation broth is removed from the culture vessel or fermentation tank, collected where appropriate, and used for providing a product containing the L-lysine, in liquid or solid form. The expression "recovering the L-lysine-containing product" is also used for this. In the simplest case, the L-lysine -containing fermentation broth itself, which has been removed from the fermentation tank, constitutes the recovered product.

An abundance of technical instructions for measures a), b), c) and d) are available in the art.

Removal of water (measure a)) can be achieved inter alia by evaporation, using e.g. a falling film evaporator, by reverse osmosis or nanofiltration. The concentrates thus obtained can be further worked up by spray drying or spray granulation. It is likewise possible to dry the fermentation broth directly using spray drying or spray granulation.

Accordingly, a method according to the invention comprises extracting or substantially eliminating water from said fermentation broth. In particular at least 40 % (w/w), preferred at least 90 % (w/w), more preferred at least 95 % (w/w) water are extracted from the fermentation broth.

Removal of the biomass (measure b)) can be achieved inter alia by centrifugation, filtration or decantation or a combination thereof.

Removal of the organic by-products (measure c)) or removal of residual components of the medium (measure d) can be achieved inter alia by chromatography, e.g. ion exchange chromatography, treatment with activated carbon or crystallization. In case the organic by-products or residual components of the medium are present in the fermentation broth as solids they can be removed by measure b).

Accordingly, the manufacturing of an L-lysine product according to the invention comprises a purification step, preferably selected from the group consisting ion exchange chromatography, treatment with activated carbon or crystallization.

Thus e. g. a product containing L-lysine x HCI, preferably containing ≥ 80 % L-lysine x HCI, particularly preferred ≥ 90 % L-lysine x HCI or ≥ 95 % L-lysine x HCI can be obtained.

General instructions on separation, purification and granulation methods can be found inter alia in the book of R. Ghosh "Principles of Bioseperation Engineering" (World Scientific Publishing, 2006), the book of F. J. Dechow "Seperation and Purification Techniques in Biotechnology" (Noyes Publications, 1989), the article "Bioseparation" of Shaeiwitz et al. (Ullmann's Encyclopedia of Industrial Chemistry, Wiley-VCH, 2012) and the book of P. Serno et al. "Granulieren" (Editio Cantor Verlag, 2007).

A downstream processing scheme for L-lysine products can be found in the article "L-lysine Production" of R. Kelle et al. (L. Eggeling and M. Bott (Handbook of Corynebacterium glutamicum, CRC Press, 2005)). US5279744 teaches the manufacturing of a purified L-lysine product by ion exchange chromatography. US5431933 teaches the manufacturing of dry L-amino acid products, e. g. an L-lysine product, containing most of the constituents of the fermentation broth.

Thus a concentration or purification of the L-lysine is achieved and a product having the desired content of said L-lysine is provided.

Analysis of L-lysine to determine its concentration at one or more time(s) during the fermentation can take place by separating the L-lysine by means of ion exchange chromatography, preferably cation exchange chromatography, with subsequent post-column derivatization using ninhydrin, as described in Spackman et al. (Analytical Chemistry 30: 1190-1206 (1958)). It is also possible to employ ortho-phthalaldehyde rather than ninhydrin for post-column derivatization. An overview article on ion exchange chromatography can be found in Pickering (LC.GC (Magazine of Chromatographic Science 7(6):484-487 (1989)). It is likewise possible to carry out a pre-column derivatization, for example using ortho-phthalaldehyde or phenyl isothiocyanate, and to fractionate the resulting amino acid derivates by reversed-phase chromatography (RP), preferably in the form of high-performance liquid chromatography (HPLC). A method of this type is described, for example, in Lindroth et al. (Analytical Chemistry 51:1167-1174 (1979)). Detection is carried out photometrically (absorption, fluorescence). A review regarding amino acid analysis can be found inter alia in the textbook "Bioanalytik" by Lottspeich and Zorbas (Spektrum Akademischer Verlag, Heidelberg, Germany 1998).

### EXPERIMENTAL SECTION

### A) MATERIALS and METHODS

The molecular biology kits, primers and chemicals used and some details of the methods applied are briefly described herewith.

### 1. Antibiotics and chemicals

a. Kanamycin: Kanamycin solution from *Streptomyces kanamyceticus* from Sigma Aldrich (St. Louis, USA, Cat. no. K0254).
b. Nalidixic acid: Nalidixic acid sodium salt from Sigma Aldrich (St. Louis, USA, Cat. no. N4382).
c. If not stated otherwise, all chemicals were purchased analytically pure from Merck (Darmstadt, Germany), Sigma Aldrich (St. Louis, USA) or Carl-Roth (Karlsruhe, Germany).

### 2. Cultivation

If not stated otherwise, all cultivation / incubation procedures were performed as follows herewith:
a. LB broth (MILLER) from Merck (Darmstadt, Germany; Cat. no. 110285) was used to cultivate *E. coli* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 37°C and 200 rpm.
b. LB agar (MILLER) from Merck (Darmstadt, Germany Cat. no. 110283) was used for cultivation of *E*. *coli* strains on agar plates. The agar plates were incubated at 37°C in an INCU-Line® mini incubator from VWR (Radnor, USA).
c. Brain heart infusion broth (BHI) from Merck (Darmstadt, Germany; Cat. no. 110493) was used to cultivate *C*. *glutamicum* strains in liquid medium. The liquid cultures (10 ml liquid medium per 100 ml Erlenmeyer flask with 3 baffles) were incubated in the Infors HT Multitron standard incubator shaker from Infors GmbH (Einsbach, Germany) at 33°C and 200 rpm.
d. Brain heart agar (BHI-agar) from Merck (Darmstadt, Germany; Cat. no. 113825) was used for cultivation of *C*. *glutamicum* strains on agar plates. The agar plates were incubated at 33°C in an incubator from Heraeus Instruments with Kelvitron® temperature controller (Hanau, Germany).

### 3. Determining optical density

a. The optical density of bacterial suspensions in shake flask cultures was determined at 600 nm (OD600) using the BioPhotometer from Eppendorf AG (Hamburg, Germany).
b. The optical density of bacterial suspensions produced in the Wouter Duetz (WDS) micro fermentation system (24-Well Plates) was determined at 660 nm (OD660) with the GENios™ plate reader from Tecan Group AG (Männedorf, Switzerland).

### 4. Centrifugation

a. Benchtop centrifuge for reaction tubes with a volume up to 2 ml
   Bacterial suspensions with a maximum volume of 2 ml were caused to sediment using 1 ml or 2 ml reaction tubes (e.g. Eppendorf Tubes® 3810X) using an Eppendorf 5417 R centrifuge (5 min. at 13.000 rpm).
b. Benchtop centrifuge for tubes with a volume up to 50 ml
   Bacterial suspensions with a maximum volume of 50 ml were caused to sediment using 15 ml or 50 ml centrifuge tubes (e.g. Falcon™ 50 ml Conical Centrifuge Tubes) using an Eppendorf 5810 R centrifuge for 10 min. at 4.000 rpm.

### 5. Polymerase chain reaction (PCR)

PCR with a proof reading (high fidelity) polymerase was used to amplify a desired segment of DNA before Sanger sequencing.
a. The Phusion® High-Fidelity DNA Polymerase Kit (NEB Phusion Kit) from New England BioLabs Inc. (Ipswich, USA; Cat. No. M0530) was used for template-correct amplification of selected DNA regions according to the instructions of the manufacturer (see table 1).

**Table 1: Thermocycling conditions for PCR with Phusion® High-Fidelity DNA Polymerase Kit from NEB Inc.**

| PCR-program | | | |
|---|---|---|---|
| Step | Time [min.] | T [°C] | Description |
| 1 | 00:30 | 98 | Initial denaturation step |
| 2 | 00:05 | 98 | Denaturation step |
| 3 | 00:30 | 60 | Annealing step |
| 4 | 00:xx | 72 | Elongation step 15-30 sec. per kb DNA |
| | | | Repeat step 2 to 4: 30 x |
| 5 | 05:00 | 72 | Final Elongation step |
| 6 | Hold | 4 | Cooling step |

### b. Primer

The oligonucleotides used were synthesized by eurofins genomics GmbH (Ebersberg, Germany) using the phosphoramidite method described by McBride and Caruthers (Tetrahedron Lett. 24, 245-248, 1983).

### c. Template

As PCR template either a suitably diluted solution of isolated plasmid DNA or of isolated total DNA from a *C*. *glutamicum* liquid culture or the total DNA contained in a colony was used (colony PCR). For said colony PCR the template was prepared by taking cell material with a toothpick from a colony on an agar plate and placing the cell material directly into the PCR reaction tube. The cell material was heated for 10 sec. with 800 W in a microwave oven type Mikrowave & Grill from SEVERIN Elektrogeräte GmbH (Sundern, Germany) and then the PCR reagents were added to the template in the PCR reaction tube.

### d. PCR Cycler

PCR's were carried out in PCR Cyclers type Mastercycler or Mastercycler nexus gradient from Eppendorf AG (Hamburg, Germany).

### 6. Detection of mutations using FRET

The presence of a given mutation, e.g. a nucleobase exchange, was detected by real-time PCR in combination with FRET hybridization probes. The term FRET is the abbreviation for fluorescence resonance energy transfer. As real-time PCR instrument a Lightcycler from Roche Diagnostics® was used (see below).

This method was e. g. used by M. J. Lay and C. T. Wittwer (Clinical Chemistry 42 (12), 2262-2267 (1997)) for the genotyping of factor V Leiden. Cyril DS Mamotte (The Clinical Biochemist Reviews 27, 63-75 (2006) reviews the genotyping of single nucleotide substitutions using this method. Summaries concerning this method may be found in the textbooks Lewin's Genes XII by Jocelyn E. Krebs, Elliott S. Goldstein and Stephan T. Kilpatrick (Jones and Bartlett Publishers, US, 2018), Molecular Diagnostics, 12 Tests that changed everything by W. Edward Highsmith (Humana Press, Springer, New York, 2014) or elsewhere in the art.

The FRET hybridization donor probe was labelled with the fluorescent dye fluorescein and the acceptor probe with the fluorescent dye LC-Red640. In essence the detection method comprised three steps: colony PCR, probe hybridization and subsequent melting curve analysis. The method is simply referred to as real-time PCR herewith.

### a. Primers and Probes

The oligonucleotides used were synthesized by eurofins genomics GmbH (Ebersberg, Germany).

### b. Template

As PCR template the total DNA contained in a colony was used. It was prepared by taking cell material with a toothpick from a colony on an agar plate and placing the cell material directly into the PCR reaction tube. The cell material was heated for 10 sec. with 800 W in a microwave oven type Mikrowave & Grill from SEVERIN Elektrogeräte GmbH (Sundern, Germany) and then the PCR reagents were added to the template in the PCR reaction tube.

### e. Reaction Mix

The Type-it® Fast SNP probe PCR Kit (Type-it Kit) from Qiagen (Hilden, Germany, Cat.No. 206045) was used for real-time detection of the mutations. Therefore 2.5 µl of the Qiagen Fast SNP Puffer (2x) was mixed with 0.5 µl of each of the LC-PCR-Primers [10 µM] and 0.5 µl of each of the 1:500 diluted acceptor and donor probe [100 pmol/µl] to get the mastermix for the real-time PCR.

**Table 2: Thermocycling conditions for PCR with the LightCycler® (step 1-3) and melting curve analysis (step 4-6).**

| PCR-program | | | |
|---|---|---|---|
| Step | Time [sec.] | T [°C] | Description |
| 1 | 15 | 95 | Denaturation step (and Activation of HotStarTaq™ DNA polymerase) |
| 2 | 05 | 55 | Annealing step |
| 3 | 30 | 72 | Elongation step |
| | | | Repeat step 1 to 3: 50 x |
| 4 | 10 | 95 | Denaturation step |
| 5 | 30 | 40 | Probe hybridisation |
| 6 | | 40- 80 | Melting curve analysis |
| 7 | | 80- 40 | Cooling |

### f. PCR Cycler

The reactions were carried out in a LightCycler® 2.0 Instrument and analysed with LightCycler® Software 4.1 of Roche Diagnostics (Rotkreuz, Switzerland).

### 7. Chemical transformation of E. coli

*E. coli* K-12 strain S17-1 was used as donor for conju-gational transfer of plasmids based on pK18mobsacB from *E. coli* to *C*. *glutamicum.* Strain S17-1 is described by Simon, R. et al. (Bio/Technology 1, 784-794, 1983). It is available from the American Type Culture Collection under the access number ATCC47055.

Chemically competent *E. coli* S17-1 cells were made as follows: A preculture of 10 ml LB medium (10 ml liquid medium per 100 ml Erlenmeyer flask with 3 baffles) was inoculated with 100 µl bacterial suspension of strain S17-1 and the culture was incubated overnight for about 18 h at 37°C and 250 rpm. The main culture (70 ml LB contained in a 250 ml Erlenmeyer flask with 3 baffles) was inoculated with 300 µl of the preculture and incubated up to an OD600 of 0.5-0.8 at 37°C. The culture was centrifuged for 6 min. at 4°C and 4000 rpm and the supernatant was discarded. The cell pellet was resuspended in 20 ml sterile, ice-cold 50 mM CaCl₂ solution and incubated on ice for 30 min.. After another centrifugation step, the pellet was resuspended in 5 ml ice-cold 50 mM CaCl₂ solution and the suspension incubated on ice for 30 min.. The cell suspension was then adjusted to a final concentration of 20 % glycerol (v/v) with 85 % (v/v) sterile ice-cold glycerol. The suspension was divided into 50 µl aliquots and stored at -80°C.

To transform S17-1 cells, the protocol according to Tang et al. (Nucleic Acids Res. 22(14), 2857-2858, 1994) with a heat shock of 45 sec. was used.

### 8. Conjugation of C. glutamicum

The pK18mobsacB plasmid system described by Schafer et al. (Gene 145, 69 - 73, 1994) was used to integrate desired DNA fragments into the chromosome of *C*. *glutamicum.* A modified conjugation method of Schafer et al. (Journal of Bacteriology 172, 1663 - 1666, 1990) was used to transfer the respective plasmid into the desired *C*. *glutamicum* recipient strain.

Liquid cultures of the *C*. *glutamicum* strains were carried out in BHI medium at 33°C. The heat shock was carried out at 48.5°C for 9 min.. Transconjugants were selected by plating the conjugation batch on EM8 agar (Table 3), which was supplemented with 25 mg/l kanamycin and 50 mg/l nalidixic acid. The EM8 agar plates were incubated for 72 h at 33°C.

**Table 3: Composition of the EM8 agar**

| Components | Concentration (g/l) |
|---|---|
| Glucose (sterile-filtered) | 23 |
| CSL (corn steep liquor; Roquette; solid content 48±2 % w/w) | 30 |
| Peptone from soymeal (Merck, Germany) | 40 |
| (NH₄)₂SO₄ | 8 |
| Urea | 3 |
| KH₂PO₄ | 4 |
| MgSO₄ · 7 H₂O | 0.5 |
| FeSO₄ · 7 H₂O | 0.01 |
| CuSO₄ · 5 H₂O | 0.001 |
| ZnSO₄ · 7 H₂O | 0.01 |
| Calcium pantothenate, D(+) | 0.01 |
| Thiamine | 0.001 |
| Inositol | 0.1 |
| Nicotinic acid | 0.001 |
| Biotin (sterile-filtered) | 0.005 |
| CaCO₃ (autoclaved separately) | 1.6 |
| Agar-Agar (Merck, Germany) | 14 |

Sterile toothpicks were used to transfer the transconjugants onto BHI agar, which was supplemented with 25 mg/l kanamycin and 50 mg/l nalidixic acid. The agar plates were incubated for 20 h at 33°C. The cultures of the respective transconjugants produced in this manner were then propagated further for 24 h at 33°C in 10 ml BHI medium contained in 100 ml Erlenmeyer flasks with 3 baffles. An aliquot was taken from the liquid culture suitably diluted and plated (typically 100 to 200 µl) on BHI agar which was supplemented with 10% saccharose. The agar plates were incubated for 48 h at 33°C. The colonies growing on the saccharose containing agar plates were then examined for the phenotype kanamycin sensitivity. To do so a toothpick was used to remove cell material from the colony and to transfer it onto BHI agar containing 25 mg/l kanamycin and onto BHI agar containing 10% saccharose. The agar plates were incubated for 60 h at 33°C. Clones that proved to be sensitive to kanamycin and resistant to saccharose were examined for integration of the desired DNA fragment by means of real-time PCR.

### 9. Determining nucleotide sequences

Nucleotide sequences of DNA molecules were determined by eurofins genomics GmbH (Ebersberg, Germany) by cycle sequencing, using the dideoxy chain termination method of Sanger et al. (Proceedings of the National Academy of Sciences USA 74, 5463 - 5467, 1977), on Applied Biosystems® (Carlsbad, CA, USA) 3730xl DNA Analyzers. Clonemanager Professional 9 software from Scientific & Educational Software (Denver, USA) was used to visualise and evaluate the sequences.

### 10. Glycerol stocks of E.coli and C. glutamicum strains

For long time storage of *E.coli-* and *C*. *glutamicum* strains glycerol stocks were prepared. Selected *E. coli* clones were cultivated in 10 ml LB medium supplemented with 2 g/l glucose. Selected *C*. *glutamicum* clones were cultivated in two fold concentrated BHI medium supplemented with 2 g/l glucose. Cultures of plasmid containing *E*. *coli* strains were supplemented with 50 mg/l kanamycin. Cultures of plasmid containing *C*. *glutamicum* strains were supplemented with 25 mg/l kanamycin. The medium was contained in 100 ml Erlenmeyer flasks with 3 baffles. It was inoculated with a loop of cells taken from a colony and the culture incubated for about 18 h at 37°C and 200 rpm in the case of *E. coli* and 33°C and 200 rpm in the case of *C*. *glutamicum.* After said incubation period 1.2 ml 85% (v/v) sterile glycerol were added to the culture. The obtained glycerol containing cell suspension was then aliquoted in 2 ml portions and stored at -80°C.

### 11. Cultivation system according to Wouter Duetz (WDS)

The millilitre-scale cultivation system according to Duetz (Trends Microbiol. 2007; 15(10):469-75) was used to investigate the performance of the *C*. *glutamicum* strains constructed. For this purpose, 24-deepwell microplates (24 well WDS plates) from EnzyScreen BV (Heemstede, Netherlands; Cat. no. CR1424), filled with 2.5 mL medium were used.
Precultures of the strains were done in 10 ml two fold concentrated BHI medium. The medium was contained in a 100 ml Erlenmeyer flask with 3 baffles. It was inoculated with 100 µl of a glycerol stock culture and the culture incubated for 24 h at 33°C and 200 rpm.
After said incubation period the optical densities OD600 of the precultures were determined.
The main cultures were done by inoculating the 2.5 ml medium containing wells of the 24 Well WDS-Plate with an aliquot of the preculture to give an optical density OD600 of 0.1.
As medium for the main culture CGXII medium described by Keilhauer et al. (J. Bacteriol. 1993 Sep; 175(17): 5595-5603) was used. For convenience the composition of the CGXII medium is shown in table 4.

**Table 4: Composition of Keilhauer's CGXII medium.**

| Components | Concentration (g/l) |
|---|---|
| MOPS (3-(N-Morpholino)propanesulfonic acid) | 42 |
| (NH₄)₂SO₄ | 20 |
| Urea | 5 |
| KH₂PO₄ | 1 |
| K₂HPO₄ | 1 |
| MgSO₄ · 7 H₂O | 0.25 |
| CaCl₂ | 0.01 |
| FeSO₄ · 7 H₂O | 0.01 |
| MnSO₄ H₂O | 0.01 |
| ZnSO₄ · 7 H₂O | 0.001 |
| CuSO₄ · 5 H₂O | 0.0002 |
| NiCl₂ 6 H₂O | 0.00002 |
| Biotin (sterile-filtered) | 0.0002 |
| Protocatechuic acid (sterile-filtered) | 0.03 |
| Carbon source (sterile-filtered) | as needed |
| adjust the pH to 7 with NaOH | |

These main cultures were incubated for approximately 45 h at 33 °C and 300 rpm in an Infors HT Multitron standard incubator shaker from Infors GmbH (Bottmingen, Switzerland) until complete consumption of glucose.
The glucose concentration in the suspension was analysed with the blood glucose-meter OneTouch Vita® from LifeScan (Johnson & Johnson Medical GmbH, Neuss, Germany).
After cultivation the culture suspensions were transferred to a deep well microplate. A part of the culture suspension was suitably diluted to measure the OD600. Another part of the culture was centrifuged and the concentration of L-amino acids, in particular L-lysine, and residual glucose were analysed in the supernatant.

### 12. Amino acid analyser

The concentration of L-lysine and other L-amino acids, e.g. L-valine, in the culture supernatants was determined by ion exchange chromatography using a SYKAM S433 amino acid analyser from SYKAM Vertriebs GmbH (Fürstenfeldbruck, Germany). As solid phase a column with spherical, polystyrene-based cation exchanger (Peek LCA N04/Na, dimension 150 x 4.6 mm) from SYKAM was used. Depending on the L-amino acid the separation takes place in an isocratic run using a mixture of buffers A and B for elution or by gradient elution using said buffers. As buffer A an aquous solution containing in 20 l 263 g trisodium citrate, 120 g citric acid, 1100 ml methanol, 100 ml 37 % HCI and 2 ml octanoic acid (final pH 3.5) was used. As buffer B an aquous solution containing in 20 I 392 g trisodium citrate, 100 g boric acidand 2 ml octanoic acid (final pH 10.2) was used. The free amino acids were coloured with ninhydrin through post-column derivatization and detected photometrically at 570 nm.

### 13. Glucose determination with continuous flow system (CFS)

A SANplus multi-channel continuous flow analyser from SKALAR analytic GmbH (Erkelenz, Germany) was used to determine the concentration of glucose in the supernatant. Glucose was detected with a coupled-enzyme assay (Hexokinase/ Glucose-6-Phosphate-Dehydrogenase) via NADH formation.

### B) EXPERIMENTAL RESULTS

### Example 1

Sequence of the NCgl1480* gene of *C*. *glutamicum* strain DM1933
The "*" in the designation NCgl1480* indicates that according to this invention the coding sequence of NCgl1480* comprises a sequence coding for 26 additional amino acids at the 5'-terminus as compared to the information given under locus_tag NCgl1480 (see SEQ ID NO:1 and SEQ ID NO:2).

Strain DM1933 is an L-lysine producer described by Blombach et al. (Applied and Environmental Microbiology 75(2), 419-427, 2009). It is deposited according to the Budapest treaty at the DSMZ under accession number DSM25442.

The nucleotide sequence of the chromosome of strain DM1933 was determined by Illumina whole-genome sequencing technology (Illumina Inc., San Diego, CA, US). See e.g. Benjak et al. (2015) Whole-Genome Sequencing for Comparative Genomics and De Novo Genome Assembly. In: Parish T., Roberts D. (eds) Mycobacteria Protocols. Methods in Molecular Biology, Vol 1285. Humana Press, NY, US) and Bennet, S. (Pharmacogenomics 5(4), 433-438, 2004).
It was found that the nucleotide sequence of the NCgl1480* coding sequence of strain DM1933 including the nucleotide sequence upstream and downstream thereof is identical to that of ATCC13032 shown in SEQ ID NO:1.

DM1933 contains in its chromosome a variant of the aspartokinase gene encoding a feedback resistant aspartokinase polypeptide. Said feedback resistant aspartokinase polypeptide has the amino acid sequence of SEQ ID NO:6 of the sequence listing, wherein the amino acid threonine (Thr) at position 311 of the amino acid sequence is replaced by isoleucine (Ile). In US 7,338,790 the abbreviation "lysC T3111" is used to indicate said exchange. Blombach et al. use the abbreviation "lysC(T311I)".

### Example 2

Construction of plasmid pK18mobsacB_NCgl1480*_Mut Plasmid pK18mobsacB_NCgl1480*_Mut was constructed to enable incorporation of the mutations causing the amino acid exchanges V90I, G159R, G449D and S454F into the nucleotide sequence of the chromosome of strain DM1933. The plasmid is based on the mobilizable vector pK18mobsacB described by Schafer et al. (Gene 145, 69-73, 1994). For the construction of pK18mobsacB_NCgl1480*_Mut the NCgl1480*_Mut sequence according to SEQ ID NO:11 was synthesized and subcloned into pK18mobsacB by GeneArt (ThermoFisher Scientific (Waltham, USA)).

To assemble the plasmid pK18mobsacB_NCgl1480*_Mut the two polynucleotides i.e. the vector pK18mobsacB cut with Sbfl and the polynucleotide NCgl1480*_Mut cut with Sbfl were ligated and the ligation mixture used to transform an appropriate *E. coli* strain (Geneart; ThermoFisher Scientific (Waltham, USA)).

DNA of plasmid pK18mobsacB_NCgl1480*_Mut was isolated from a transformant.

### Example 3

Construction of strain DM1933_NCgl1480*_Mut
The plasmid pK18mobsacB_NCgl1480*_Mut obtained in example 2 was used to incorporate the mutations leading to the amino acid exchanges V90I, G159R, G449D and S454F (see nucleotide positions 1190, 1397, 2268 and 2283 of SEQ ID NO:3) into the chromosome of the L-lysine producer DM1933.

Chemically competent cells of *E*. *coli* strain S17-1 were transformed with plasmid DNA of pK18mobsacB_NCgl1480*_Mut. The modified conjugation method of Schafer et al. (Journal of Bacteriology 172, 1663 - 1666, 1990) as described in materials and methods was used for conjugal transfer into the strain DM1933 and for selection of transconjugant clones by virtue of their saccharose resistance and kanamycin sensitivity phenotype.

Transconjugant clones were analyzed by two separate real-time PCRs to detect the mutations leading to the amino acid exchanges V90I and S454F using the Type-it Kit. To detect the mutation leading to the amino acid exchange V90I the primers LC-NCgl1480_1 and LC-NCgl1480_2 for PCR amplification and NCgl1480_90_C as acceptor probe and NCgl1480_90_A as donor probe for melting curve analysis were used (table 5). To detect the mutation leading to the amino acid exchange S454F the primers LC-NCgl1480_3 and LC-NCgl1480_4 for PCR amplification and NCgl1480_454_C as acceptor probe and NCgl1480_454_A as donor probe for melting curve analysis were used (table 5).

**Table 5: List of primers and probes used for real-time PCR.**

| name | sequence |
|---|---|
| LC-NCgl1480_1 | AGCGGAGCTTTGCTTAGTTC |
| LC-NCgl1480_2 | TGCTTCGATCTGCGCTTGAG |
| NCgl1480_90_C¹ | CCAGCTCGATGCGGTTAATT |
| NCgl1480_90_A² | AGGGACTTGTTCACTTCTTCACGCAGAGCACCCAT |
| LC-NCgl1480_3 | CGCTGCGGCTGCTCTAATTG |
| LC-NCgl1480_4 | GCGTACTGCACACCCAACTG |
| NCgl1480_454_C¹ | GTTGCTCTGGAAATCTCCAG |
| NCgl1480_454_A² | CTGTGGAATCGTTGCCTGTGGAATCGTTGTCGCTG |

| | |
|---|---|
| ¹ acceptor probe labelled with LC-Red640 at the 5'-end and phosphorylated at the 3'-end ² donor probe labelled with fluorescein at the 3'-end | |

One of the transconjugant clones thus characterized was called DM1933_NCgl1480*_Mut. The nucleotide sequence of the chromosomal region of strain DM1933_NCgl1480*_Mut containing the mutated nucleotide sequence was analyzed by Sanger sequencing.

For this purpose a PCR amplificate was produced spanning the site of mutations. A colony PCR was done using the primers LC-NCgl1480_1 and LC-NCgl1480_4 (see table 5) and the NEB Phusion Kit with an elongation time of 45 sec. (table 1, step 4). The amplificate obtained was then sequenced using the same primers (LC-NCgl1480_1 and LC-NCgl1480_4).
The nucleotide sequences of the primers and probes used in this context (see table 5) are also shown in SEQ ID NO:12 to 19 of the sequence listing.

The nucleotide sequence obtained is shown in SEQ ID NO:20. The result showed that strain DM1933_NCgl1480*_Mut contained all desired mutations, or the desired mutated nucleotide sequence resp., in its chromosome with the effect of the amino acid exchanges V90I, G159R, G449D and S454F.

A glycerol stock culture of the transconjugant clone was prepared and used as starting material for further investigations.

### Example 4

L-lysine production by strain DM1933_NCgl1480*_Mut
Strains DM1933 (reference) and DM1933_NCgl1480*_Mut obtained in example 3 were analyzed for their ability to produce L-lysine from glucose by batch cultivation using the cultivation system according to Wouter Duetz.
As medium CGXII containing 20 g/l glucose as carbon source was used. The cultures were incubated for 45 h until complete consumption of glucose as confirmed by glucose analysis using blood glucose-meter and the concentrations of L-lysine and the optical density OD660 were determined. The result of the experiment is presented in table 7.

**Table 7: L-lysine production by strain DM1933_NCgl1480*_Mut.**

| strain | L-lysine¹ (g/l) | OD660 |
|---|---|---|
| DM1933 | 3.7 | 9.5 |
| DM1933_ NCgl1480*_Mut | 4.1 | 8.9 |

| | | |
|---|---|---|
| ¹ as L-lysine x HCI | | |

The experiment shows that L-lysine production was increased in strain DM1933_NCgl1480*_Mut as compared to the parent strain DM1933.

## Claims

1. A method for the fermentative production of L-lysine comprising the steps of
a) providing a bacterium of the species *Corynebacterium glutamicum* having the ability to excrete L-lysine containing in its chromosome a polynucleotide encoding a polypeptide comprising the amino acid sequence of SEQ ID NO:4,
b) cultivating the bacterium in a suitable medium under suitable conditions, and
c) accumulating said L-lysine in the medium to form an L-lysine containing fermentation broth.

2. The method as claimed in claim 1, wherein in the bacterium provided said polynucleotide encoding said amino acid sequence comprises the nucleotide sequence of SEQ ID NO:3 positions 923 to 2812.

3. The method as claimed in claim 1, wherein in the bacterium provided the polynucleotide encoding said amino acid sequence comprises the nucleotide sequence of SEQ ID NO:3 positions 923 to 2815.

## Patentansprüche

1. Verfahren zur fermentativen Herstellung von L-Lysin, umfassend die Schritte
a) Bereitstellen eines Bakteriums der Spezies *Corynebacterium glutamicum* mit der Fähigkeit, L-Lysin auszuscheiden, dessen Chromosom ein Polynukleotid enthält, das ein Polypeptid codiert, das die Aminosäuresequenz unter SEQ ID NO:4 umfasst,
b) Kultivieren des Bakteriums in einem geeigneten Medium unter geeigneten Bedingungen und
c) Anreichern des L-Lysins im Medium unter Bildung einer L-Lysin enthaltenden Fermentationsbrühe.

2. Verfahren nach Anspruch 1, wobei im bereitgestellten Bakterium das die Aminosäuresequenz codierende Polynukleotid Positionen 923 bis 2812 der Nukleotidsequenz unter SEQ ID NO:3 umfasst.

3. Verfahren nach Anspruch 1, wobei im bereitgestellten Bakterium das die Aminosäuresequenz codierende Polynukleotid Positionen 923 bis 2815 der Nukleotidsequenz unter SEQ ID NO:3 umfasst.

## Revendications

1. Procédé pour la production par fermentation de L-lysine comprenant les étapes de
a) mise à disposition d'une bactérie de l'espèce *Corynebacterium glutamicum* possédant la capacité de sécréter la L-lysine, contenant dans son chromosome un polynucléotide codant pour un polypeptide comprenant la séquence d'acides aminés de SEQ ID N° : 4,
b) culture de la bactérie dans un milieu approprié dans des conditions appropriées, et
c) accumulation de ladite L-lysine dans le milieu pour former un bouillon de fermentation contenant de la L-lysine.

2. Procédé selon la revendication 1, dans lequel, dans la bactérie mise à disposition, ledit polynucléotide codant pour ladite séquence d'acides aminés comprend la séquence de nucléotides des positions 923 à 2 812 de SEQ ID N° : 3.

3. Procédé selon la revendication 1, dans lequel, dans la bactérie mise à disposition, ledit polynucléotide codant pour ladite séquence d'acides aminés comprend la séquence de nucléotides des positions 923 à 2 815 de SEQ ID N° : 3.


### SEQUENCE LISTING

<110> Evonik Degussa GmbH
<120> Method for the fermentative production of L-Lysine
<130> file: 2018P00198EP
<140> EP18185087.6
   <141> 2018-07-24
<160> 20
<170> PatentIn version 3.5
<210> 1
   <211> 3815
   <212> DNA
   <213> Corynebacterium glutamicum ATCC13032
<220>
   <221> misc_feature
   <222> (1)..(411)
   <223> 3'-sequence of the cds of NCgl1481
<220>
   <221> misc_feature
   <222> (412)..(413)
   <223> taa stop codon
<220>
   <221> misc_feature
   <222> (565)..(565)
   <223> nucleobase cytosine
<220>
   <221> misc_feature
   <222> (585)..(585)
   <223> nucleobase guanine
<220>
   <221> misc_feature
   <222> (632)..(637)
   <223> taatgt motif according to Pfeifer-Sancar et al.
<220>
   <221> misc_feature
   <222> (644)..(644)
   <223> transcriptional start site according to Pfeifer-Sancar et al.
<220>
   <221> CDS
   <222> (923)..(2812)
   <223> coding sequence
<220>
   <221> misc_feature
   <222> (1001)..(2812)
   <223> coding sequence according to NCgl1480
<220>
   <221> misc_feature
   <222> (1190)..(1192)
   <223> gtc codon for valine
<220>
   <221> misc_feature
   <222> (1397)..(1399)
   <223> ggg codon for glycine
<220>
   <221> misc_feature
   <222> (2267)..(2269)
   <223> ggt codon for glycine
<220>
   <221> misc_feature
   <222> (2282)..(2284)
   <223> tcc codon for serine
<220>
   <221> misc_feature
   <222> (2813)..(2815)
   <223> tag stop codon
<220>
   <221> misc_feature
   <222> (2889)..(3815)
   <223> 5'-sequence of the cds of NCgl1479
<400> 1
   tttccgactt tggaaaaaga ccttattaat gttgtgacag atgcaaagga atcagatttt        60
   ggatctttag gggtcgtgat tcttgatgaa actccagtga tgacttccaa cctgagggat       120
   attgcgcagg agctgttgat tcaaacggat ctggacaccg ttgtggtgcg ggctccaatg       180
   tcggctgcgg tggtgagtga tgttcattcg agggcggcgc ttgagtcagg gcagcatgat       240
   ttgcttggga ccactgatta cgtgctggga acggagcttt tggtacagga tgtaacggaa       300
   tcgacggtgg ggaatattga ttggggtcaa ttgctgattt gggggttggt tgctttggca       360
   atcgccgtgg ttgttgcggg tgcgtctgtg cgtcgaaaag caatatcttt ataagtaaag       420
   ttctaaagct ttacttatag cactttcggt aggtgtcgag aaaattccca aattgcggaa       480
   aattcacata ctttgtgcca caaagtgata aacatcacaa aatttcatga ctcagccatt       540
   catgcaggcc agaacatgca aaaccttgca cgctctggcc ttttgttatg taactgttgt       600
   gaaagatgta tcaaaagtta ccaaggtgac ttaatgttca gatatcgact catgggttgt       660
   tttccaaaac ttgaatcaat ttgagtaaca gtagttatca agcgttaaac cttgaaactt       720
   ccactctttt tactgatggg catttgggca aacggggaag cttgctggag atgcaataaa       780
   tcggtgaatg gttggtgcaa tgaagaggag agtggcccat agtttccgct acggaacgcg       840
   gagtctttac gtttcaattc ttggcaagta tctagcgtca aggcttgata tgtgaaggga       900
   ttcggattga acaggagaac at gtg gcc aat cta gat tca gtt cgc cct ggg        952
                            Met Ala Asn Leu Asp Ser Val Arg Pro Gly
                            1               5                   10
   gtg cgg gca gct ctt cga cgt acc cat acc cag gta aaa ggt gtc acg        1000
   Val Arg Ala Ala Leu Arg Arg Thr His Thr Gln Val Lys Gly Val Thr
                   15                  20                  25
   ttg acc agg gcg ttg att gcg ctt gca gta agc gga gct ttg ctt agt        1048
   Leu Thr Arg Ala Leu Ile Ala Leu Ala Val Ser Gly Ala Leu Leu Ser
               30                  35                  40
   tcc atg act ccg gcg gtg gcg cag cca cag aat ccg gat gac gca gcc        1096
   Ser Met Thr Pro Ala Val Ala Gln Pro Gln Asn Pro Asp Asp Ala Ala
           45                  50                  55
   att gca cag gca gag gaa aat gtt tcg gcg ggc gat ggg gaa gtc gcc        1144
   Ile Ala Gln Ala Glu Glu Asn Val Ser Ala Gly Asp Gly Glu Val Ala
       60                  65                  70
   cgc ctg gca gga tct ttg tcc agc act gac gcg gaa att aac cgc gtc        1192
   Arg Leu Ala Gly Ser Leu Ser Ser Thr Asp Ala Glu Ile Asn Arg Val
   75                  80                  85                  90
   gag ctg gaa atg ggt gct ctg cgt gaa gaa gtg aac aag tcc ctc gtg        1240
   Glu Leu Glu Met Gly Ala Leu Arg Glu Glu Val Asn Lys Ser Leu Val
                   95                  100                 105
   gat ttg cat gat gcg cag gca atc gcc gag cag gcc cgc caa gat gca        1288
   Asp Leu His Asp Ala Gln Ala Ile Ala Glu Gln Ala Arg Gln Asp Ala
               110                 115                 120
   ctt gca gcc aag aag gat ctc gat gat tct caa gcg cag atc gaa gca        1336
   Leu Ala Ala Lys Lys Asp Leu Asp Asp Ser Gln Ala Gln Ile Glu Ala
           125                 130                 135
   gcc caa gag cgc ctt gat gag att tca cgt gca gcg tat cgc caa aac        1384
   Ala Gln Glu Arg Leu Asp Glu Ile Ser Arg Ala Ala Tyr Arg Gln Asn
       140                 145                 150
   gga acc tcc aag ggg ctt tca ggc atc tcg ggc aat gga aat tct gaa        1432
   Gly Thr Ser Lys Gly Leu Ser Gly Ile Ser Gly Asn Gly Asn Ser Glu
   155                 160                 165                 170
   gat gcg cta gat cgt cag act tac ctg cga acc agt gcg gaa aag cag        1480
   Asp Ala Leu Asp Arg Gln Thr Tyr Leu Arg Thr Ser Ala Glu Lys Gln
                   175                 180                 185
   cag gca gct gtt gaa gag ctt gat cgc ctc cgt acg gaa aac gcc aac        1528
   Gln Ala Ala Val Glu Glu Leu Asp Arg Leu Arg Thr Glu Asn Ala Asn
               190                 195                 200
   aag gaa tcg gtg ttg cgc cag gcc cgc atc gtt gct gag cag cgt gag        1576
   Lys Glu Ser Val Leu Arg Gln Ala Arg Ile Val Ala Glu Gln Arg Glu
           205                 210                 215
   gcg gaa gcc gtc gaa aag caa gtc cag acc gag gct gca att gcc gca        1624
   Ala Glu Ala Val Glu Lys Gln Val Gln Thr Glu Ala Ala Ile Ala Ala
       220                 225                 230
   aac agc gag cag ctc aat gtc ttg act aac aat cgc agt acc ttg gtt        1672
   Asn Ser Glu Gln Leu Asn Val Leu Thr Asn Asn Arg Ser Thr Leu Val
   235                 240                 245                 250
   gcc cag cgt gat ggg gct gag cgc aac ttg gcc atc gct cgt gcg cag        1720
   Ala Gln Arg Asp Gly Ala Glu Arg Asn Leu Ala Ile Ala Arg Ala Gln
                   255                 260                 265
   gcg gat aat ctg caa ggt cag cgt gct gag tac gag gaa ttc cag cag 1768
   Ala Asp Asn Leu Gln Gly Gln Arg Ala Glu Tyr Glu Glu Phe Gln Gln
   270 275 280
   gca gag cag gct cgc atc cag gcg gaa gcg gaa gct cag gct gct gcg 1816
   Ala Glu Gln Ala Arg Ile Gln Ala Glu Ala Glu Ala Gln Ala Ala Ala
   285 290 295
   gag gag aag cgt cgt gcc gat gag gct gct gca cag gca gcc gct gaa 1864
   Glu Glu Lys Arg Arg Ala Asp Glu Ala Ala Ala Gln Ala Ala Ala Glu
   300 305 310
   gct caa gaa gct gcc cag caa gct cag gcg gcg gag gaa gcc caa gcc 1912
   Ala Gln Glu Ala Ala Gln Gln Ala Gln Ala Ala Glu Glu Ala Gln Ala
   315 320 325 330
   gcg caa gca gct gag aca gca caa gcc caa gcc gcg caa gct gcg gaa 1960
   Ala Gln Ala Ala Glu Thr Ala Gln Ala Gln Ala Ala Gln Ala Ala Glu
   335 340 345
   acc caa gct gca caa gcc gcg caa gct cag gca gaa gcg aat gat cgt 2008
   Thr Gln Ala Ala Gln Ala Ala Gln Ala Gln Ala Glu Ala Asn Asp Arg
   350 355 360
   gcc gcc gcg caa cag cgt gct gca gag gct caa gca gca gcg gaa cag 2056
   Ala Ala Ala Gln Gln Arg Ala Ala Glu Ala Gln Ala Ala Ala Glu Gln
   365 370 375
   gcg caa cgt gag gct gac gct cag gcg gcc aac gat gcc caa gct cag 2104
   Ala Gln Arg Glu Ala Asp Ala Gln Ala Ala Asn Asp Ala Gln Ala Gln
   380 385 390
   gca ctg cgt gaa cag gcg ctc acc gca gcc tcc atc gct gcg gct gct 2152
   Ala Leu Arg Glu Gln Ala Leu Thr Ala Ala Ser Ile Ala Ala Ala Ala
   395 400 405 410
   cta att gcg gcg agc cag tcc agc cat gcc act act caa aat cct tac 2200
   Leu Ile Ala Ala Ser Gln Ser Ser His Ala Thr Thr Gln Asn Pro Tyr
   415 420 425
   cca act gat gaa gac gcg gat ccg acc gat att gcg gac atc caa ggc 2248
   Pro Thr Asp Glu Asp Ala Asp Pro Thr Asp Ile Ala Asp Ile Gln Gly
   430 435 440
   cca acg cag cca ggt acg ggt gag tct gga gat tcc cag agc aac tcc 2296
   Pro Thr Gln Pro Gly Thr Gly Glu Ser Gly Asp Ser Gln Ser Asn Ser
   445 450 455
   agc gac aac gat tcc aca ggc aac gat tcc aca ggc tct gac tct tca 2344
   Ser Asp Asn Asp Ser Thr Gly Asn Asp Ser Thr Gly Ser Asp Ser Ser
   460 465 470
   gat tca gat tcc tcc ggc aac gat tct tca gag gtt att tcc ggc gat 2392
   Asp Ser Asp Ser Ser Gly Asn Asp Ser Ser Glu Val Ile Ser Gly Asp
   475 480 485 490
   cgt tcc gct cag att gag act gtg att gcg cgc gcc atg agc cag ttg 2440
   Arg Ser Ala Gln Ile Glu Thr Val Ile Ala Arg Ala Met Ser Gln Leu
   495 500 505
   ggt gtg cag tac gca tgg ggt ggc ggt aac gct aat ggc cca act ctg 2488
   Gly Val Gln Tyr Ala Trp Gly Gly Gly Asn Ala Asn Gly Pro Thr Leu
   510 515 520
   ggt atc cgt gac ggt ggc gtg gcg gac tct tac ggc gat tac aac aag 2536
   Gly Ile Arg Asp Gly Gly Val Ala Asp Ser Tyr Gly Asp Tyr Asn Lys
   525 530 535
   gtt ggc ttc gac tgc tct gga ctg acc ttg tat gcg ttt gcg ggt gtg 2584
   Val Gly Phe Asp Cys Ser Gly Leu Thr Leu Tyr Ala Phe Ala Gly Val
   540 545 550
   gga att tca ctt cct cac tac acg ggc tac cag tac cag cac ggc acc 2632
   Gly Ile Ser Leu Pro His Tyr Thr Gly Tyr Gln Tyr Gln His Gly Thr
   555 560 565 570
   aag gtg tcg cct tct gag atg caa cgt ggc gat ctg atc ttc tat ggt 2680
   Lys Val Ser Pro Ser Glu Met Gln Arg Gly Asp Leu Ile Phe Tyr Gly
   575 580 585
   ccg gga gcg tct cag cac gtg gca att tac ctc ggt gat ggt cag atg 2728
   Pro Gly Ala Ser Gln His Val Ala Ile Tyr Leu Gly Asp Gly Gln Met
   590 595 600
   att gag gct ccg aat tcg ggt tct gtc gtg aag att tct cct gtt cgc 2776
   Ile Glu Ala Pro Asn Ser Gly Ser Val Val Lys Ile Ser Pro Val Arg
   605 610 615
   tgg agc gga atg acc gag agc gtg gta cgc ctc att tagtttcctc 2822
   Trp Ser Gly Met Thr Glu Ser Val Val Arg Leu Ile
   620 625 630
   ctatgaatct tgatgtggtt catgcgtttt tatgcaatat caaccaaaag ttggtacgat 2882
   cctcatatga atgaacgcac atcggatgca tttgacgccc tccttgtgct ctccttcggt 2942
   ggtcccgaag ggcacgagga ggttcgtccg tttttggaga atgtcactca cggaaggggg 3002
   attccgccgg aacgtctaga tgaagtggcg gttcattacc accacttcgg tggtatcagc 3062
   cccatcaatg cgctgaacag ggaaattatc gccaatgtgg aaaaagaatt ggcgtctcgc 3122
   gatcacaagc tgcctgttta ttttggtaac cgcaactgga agccgtttga taatgaggcc 3182
   gctgaacaaa tggctgatga cggcgtgaaa aacgcgctgg tgttggcaac ttccgcttgg 3242
   ggtggctact ccggttgtcg gcagtaccag gaagatattc agggcatgat caagcacctg 3302
   gagtctcagg ggcagtcgat cacgttcacc aagctgcgtc agttctacga tcaccctcgt 3362
   tttgtctcca ccatggctca attggttcag gattcctacg cgaagcttcc cgatgagctg 3422
   cgagatgagg cgcgtctggt cttcaccgcg cactccattc cactgactgc ggacaatgct 3482
   gcgggaaccc ctgaggatgg ctccttgtat tccacacagg tcaaggaagc gtcagcactg 3542
   attgctgagg ctgttggtgt gtcagatttt gatgtggtgt ggcagtcccg ctcgggtagc 3602
   ccgcacactc cgtggctgga gcctgacatc gtggatcacg cagtggagct caacgagaag 3662
   ggtcaaaaag cgctcgttgt ctgccctgta ggctttattt ctgatcatat ggaagtcatt 3722
   tgggatcttg attccgagct gatggaagaa gccgagaagc gcaacatggt ggtcgagcgt 3782
   gtcgctaccg ttggccccac cgatgaattc gca 3815
<210> 2
   <211> 630
   <212> PRT
   <213> Corynebacterium glutamicum ATCC13032
<400> 2
   Met Ala Asn Leu Asp Ser Val Arg Pro Gly Val Arg Ala Ala Leu Arg
   1               5                   10                  15
   Arg Thr His Thr Gln Val Lys Gly Val Thr Leu Thr Arg Ala Leu Ile
               20                  25                  30
   Ala Leu Ala Val Ser Gly Ala Leu Leu Ser Ser Met Thr Pro Ala Val
           35                  40                  45
   Ala Gln Pro Gln Asn Pro Asp Asp Ala Ala Ile Ala Gln Ala Glu Glu
       50                  55                  60
   Asn Val Ser Ala Gly Asp Gly Glu Val Ala Arg Leu Ala Gly Ser Leu
   65                  70                  75                  80
   Ser Ser Thr Asp Ala Glu Ile Asn Arg Val Glu Leu Glu Met Gly Ala
                   85                  90                  95
   Leu Arg Glu Glu Val Asn Lys Ser Leu Val Asp Leu His Asp Ala Gln
               100                 105                 110
   Ala Ile Ala Glu Gln Ala Arg Gln Asp Ala Leu Ala Ala Lys Lys Asp
           115                 120                 125
   Leu Asp Asp Ser Gln Ala Gln Ile Glu Ala Ala Gln Glu Arg Leu Asp
       130                 135                 140
   Glu Ile Ser Arg Ala Ala Tyr Arg Gln Asn Gly Thr Ser Lys Gly Leu
   145                 150                 155                 160
   Ser Gly Ile Ser Gly Asn Gly Asn Ser Glu Asp Ala Leu Asp Arg Gln
                   165                 170                 175
   Thr Tyr Leu Arg Thr Ser Ala Glu Lys Gln Gln Ala Ala Val Glu Glu
               180                 185                 190
   Leu Asp Arg Leu Arg Thr Glu Asn Ala Asn Lys Glu Ser Val Leu Arg
           195                 200                 205
   Gln Ala Arg Ile Val Ala Glu Gln Arg Glu Ala Glu Ala Val Glu Lys
       210                 215                 220
   Gln Val Gln Thr Glu Ala Ala Ile Ala Ala Asn Ser Glu Gln Leu Asn
   225                 230                 235                 240
   Val Leu Thr Asn Asn Arg Ser Thr Leu Val Ala Gln Arg Asp Gly Ala
                   245                 250                 255
   Glu Arg Asn Leu Ala Ile Ala Arg Ala Gln Ala Asp Asn Leu Gln Gly
               260                 265                 270
   Gln Arg Ala Glu Tyr Glu Glu Phe Gln Gln Ala Glu Gln Ala Arg Ile
           275                 280                 285
   Gln Ala Glu Ala Glu Ala Gln Ala Ala Ala Glu Glu Lys Arg Arg Ala
       290                 295                 300
   Asp Glu Ala Ala Ala Gln Ala Ala Ala Glu Ala Gln Glu Ala Ala Gln
   305                 310                 315                 320
   Gln Ala Gln Ala Ala Glu Glu Ala Gln Ala Ala Gln Ala Ala Glu Thr
                   325                 330                 335
   Ala Gln Ala Gln Ala Ala Gln Ala Ala Glu Thr Gln Ala Ala Gln Ala
               340                 345                 350
   Ala Gln Ala Gln Ala Glu Ala Asn Asp Arg Ala Ala Ala Gln Gln Arg
           355                 360                 365
   Ala Ala Glu Ala Gln Ala Ala Ala Glu Gln Ala Gln Arg Glu Ala Asp
       370                 375                 380
   Ala Gln Ala Ala Asn Asp Ala Gln Ala Gln Ala Leu Arg Glu Gln Ala
   385                 390                 395                 400
   Leu Thr Ala Ala Ser Ile Ala Ala Ala Ala Leu Ile Ala Ala Ser Gln
                   405                 410                 415
   Ser Ser His Ala Thr Thr Gln Asn Pro Tyr Pro Thr Asp Glu Asp Ala
               420                 425                 430
   Asp Pro Thr Asp Ile Ala Asp Ile Gln Gly Pro Thr Gln Pro Gly Thr
           435                 440                 445
   Gly Glu Ser Gly Asp Ser Gln Ser Asn Ser Ser Asp Asn Asp Ser Thr
       450                 455                 460
   Gly Asn Asp Ser Thr Gly Ser Asp Ser Ser Asp Ser Asp Ser Ser Gly
   465                 470                 475                 480
   Asn Asp Ser Ser Glu Val Ile Ser Gly Asp Arg Ser Ala Gln Ile Glu
                   485                 490                 495
   Thr Val Ile Ala Arg Ala Met Ser Gln Leu Gly Val Gln Tyr Ala Trp
               500                 505                 510
   Gly Gly Gly Asn Ala Asn Gly Pro Thr Leu Gly Ile Arg Asp Gly Gly
           515                 520                 525
   Val Ala Asp Ser Tyr Gly Asp Tyr Asn Lys Val Gly Phe Asp Cys Ser
       530                 535                 540
   Gly Leu Thr Leu Tyr Ala Phe Ala Gly Val Gly Ile Ser Leu Pro His
   545                 550                 555                 560
   Tyr Thr Gly Tyr Gln Tyr Gln His Gly Thr Lys Val Ser Pro Ser Glu
                   565                 570                 575
   Met Gln Arg Gly Asp Leu Ile Phe Tyr Gly Pro Gly Ala Ser Gln His
               580                 585                 590
   Val Ala Ile Tyr Leu Gly Asp Gly Gln Met Ile Glu Ala Pro Asn Ser
           595                 600                 605
   Gly Ser Val Val Lys Ile Ser Pro Val Arg Trp Ser Gly Met Thr Glu
       610                 615                 620
   Ser Val Val Arg Leu Ile
   625                 630
<210> 3
   <211> 3815
   <212> DNA
   <213> Corynebacterium glutamicum
<220>
   <221> misc_feature
   <222> (1)..(411)
   <223> 3'-sequence of the cds of NCgl1481
<220>
   <221> misc_feature
   <222> (412)..(414)
   <223> taa stop codon
<220>
   <221> misc_feature
   <222> (565)..(565)
   <223> nucleobase thymine
<220>
   <221> misc_feature
   <222> (585)..(585)
   <223> nucleobase guanine
<220>
   <221> misc_feature
   <222> (632)..(637)
   <223> taatgt motif according to Pfeifer-Sancar et al.
<220>
   <221> misc_feature
   <222> (644)..(644)
   <223> transcriptional start site according to Pfeifer-Sancar et al.
<220>
   <221> CDS
   <222> (923)..(2812)
   <223> coding sequence
<220>
   <221> misc_feature
   <222> (1001)..(2812)
   <223> coding sequence of a variant of NCgl1480
<220>
   <221> misc_feature
   <222> (1190)..(1192)
   <223> atc codon for isoleucine
<220>
   <221> misc_feature
   <222> (1397)..(1399)
   <223> agg codon for arginine
<220>
   <221> misc_feature
   <222> (2267)..(2269)
   <223> gat codon for aspartic acid
<220>
   <221> misc_feature
   <222> (2282)..(2284)
   <223> ttc codon for phenylalanine
<220>
   <221> misc_feature
   <222> (2813)..(2815)
   <223> tag stop codon
<220>
   <221> misc_feature
   <222> (2889)..(3815)
   <223> 5'-sequence of the cds of NCgl1479
<400> 3
   tttccgactt tggaaaaaga ccttattaat gttgtgacag atgcaaagga atcagatttt       60
   ggatctttag gggtcgtgat tcttgatgaa actccagtga tgacttccaa cctgagggat      120
   attgcgcagg agctgttgat tcaaacggat ctggacaccg ttgtggtgcg ggctccaatg      180
   tcggctgcgg tggtgagtga tgttcattcg agggcggcgc ttgagtcagg gcagcatgat      240
   ttgcttggga ccactgatta cgtgctggga acggagcttt tggtacagga tgtaacggaa      300
   tcgacggtgg ggaatattga ttggggtcaa ttgctgattt gggggttggt tgctttggca      360
   atcgccgtgg ttgttgcggg tgcgtctgtg cgtcgaaaag caatatcttt ataagtaaag      420
   ttctaaagct ttacttatag cactttcggt aggtgtcgag aaaattccca aattgcggaa      480
   aattcacata ctttgtgcca caaagtgata aacatcacaa aatttcatga ctcagccatt      540
   catgcaggcc agaacatgca aaactttgca cgctctggcc ttttgttatg taactgttgt      600
   gaaagatgta tcaaaagtta ccaaggtgac ttaatgttca gatatcgact catgggttgt      660
   tttccaaaac ttgaatcaat ttgagtaaca gtagttatca agcgttaaac cttgaaactt      720
   ccactctttt tactgatggg catttgggca aacggggaag cttgctggag atgcaataaa      780
   tcggtgaatg gttggtgcaa tgaagaggag agtggcccat agtttccgct acggaacgcg      840
   gagtctttac gtttcaattc ttggcaagta tctagcgtca aggcttgata tgtgaaggga      900
   ttcggattga acaggagaac at gtg gcc aat cta gat tca gtt cgc cct ggg       952
                            Met Ala Asn Leu Asp Ser Val Arg Pro Gly
                            1               5                   10
   gtg cgg gca gct ctt cga cgt acc cat acc cag gta aaa ggt gtc acg       1000
   Val Arg Ala Ala Leu Arg Arg Thr His Thr Gln Val Lys Gly Val Thr
                   15                  20                  25
   ttg acc agg gcg ttg att gcg ctt gca gta agc gga gct ttg ctt agt       1048
   Leu Thr Arg Ala Leu Ile Ala Leu Ala Val Ser Gly Ala Leu Leu Ser
               30                  35                  40
   tcc atg act ccg gcg gtg gcg cag cca cag aat ccg gat gac gca gcc       1096
   Ser Met Thr Pro Ala Val Ala Gln Pro Gln Asn Pro Asp Asp Ala Ala
           45                  50                  55
   att gca cag gca gag gaa aat gtt tcg gcg ggc gat ggg gaa gtc gcc       1144
   Ile Ala Gln Ala Glu Glu Asn Val Ser Ala Gly Asp Gly Glu Val Ala
       60                  65                  70
   cgc ctg gca gga tct ttg tcc agc act gac gcg gaa att aac cgc atc       1192
   Arg Leu Ala Gly Ser Leu Ser Ser Thr Asp Ala Glu Ile Asn Arg Ile
   75                  80                  85                  90
   gag ctg gaa atg ggt gct ctg cgt gaa gaa gtg aac aag tcc ctc gtg       1240
   Glu Leu Glu Met Gly Ala Leu Arg Glu Glu Val Asn Lys Ser Leu Val
                   95                  100                 105
   gat ttg cat gat gcg cag gca atc gcc gag cag gcc cgc caa gat gca       1288
   Asp Leu His Asp Ala Gln Ala Ile Ala Glu Gln Ala Arg Gln Asp Ala
               110                 115                 120
   ctt gca gcc aag aag gat ctc gat gat tct caa gcg cag atc gaa gca       1336
   Leu Ala Ala Lys Lys Asp Leu Asp Asp Ser Gln Ala Gln Ile Glu Ala
           125                 130                 135
   gcc caa gag cgc ctt gat gag att tca cgt gca gcg tat cgc caa aac       1384
   Ala Gln Glu Arg Leu Asp Glu Ile Ser Arg Ala Ala Tyr Arg Gln Asn
       140                 145                 150
   gga acc tcc aag agg ctt tca ggc atc tcg ggc aat gga aat tct gaa          1432
   Gly Thr Ser Lys Arg Leu Ser Gly Ile Ser Gly Asn Gly Asn Ser Glu
   155                 160                 165                 170
   gat gcg cta gat cgt cag act tac ctg cga acc agt gcg gaa aag cag          1480
   Asp Ala Leu Asp Arg Gln Thr Tyr Leu Arg Thr Ser Ala Glu Lys Gln
                   175                 180                 185
   cag gca gct gtt gaa gag ctt gat cgc ctc cgt acg gaa aac gcc aac          1528
   Gln Ala Ala Val Glu Glu Leu Asp Arg Leu Arg Thr Glu Asn Ala Asn
               190                 195                 200
   aag gaa tcg gtg ttg cgc cag gcc cgc atc gtt gct gag cag cgt gag          1576
   Lys Glu Ser Val Leu Arg Gln Ala Arg Ile Val Ala Glu Gln Arg Glu
           205                 210                 215
   gcg gaa gcc gtc gaa aag caa gtc cag acc gag gct gca att gcc gca          1624
   Ala Glu Ala Val Glu Lys Gln Val Gln Thr Glu Ala Ala Ile Ala Ala
       220                 225                 230
   aac agc gag cag ctc aat gtc ttg act aac aat cgc agt acc ttg gtt          1672
   Asn Ser Glu Gln Leu Asn Val Leu Thr Asn Asn Arg Ser Thr Leu Val
   235                 240                 245                 250
   gcc cag cgt gat ggg gct gag cgc aac ttg gcc atc gct cgt gcg cag          1720
   Ala Gln Arg Asp Gly Ala Glu Arg Asn Leu Ala Ile Ala Arg Ala Gln
                   255                 260                 265
   gcg gat aat ctg caa ggt cag cgt gct gag tac gag gaa ttc cag cag          1768
   Ala Asp Asn Leu Gln Gly Gln Arg Ala Glu Tyr Glu Glu Phe Gln Gln
               270                 275                 280
   gca gag cag gct cgc atc cag gcg gaa gcg gaa gct cag gct gct gcg          1816
   Ala Glu Gln Ala Arg Ile Gln Ala Glu Ala Glu Ala Gln Ala Ala Ala
           285                 290                 295
   gag gag aag cgt cgt gcc gat gag gct gct gca cag gca gcc gct gaa          1864
   Glu Glu Lys Arg Arg Ala Asp Glu Ala Ala Ala Gln Ala Ala Ala Glu
       300                 305                 310
   gct caa gaa gct gcc cag caa gct cag gcg gcg gag gaa gcc caa gcc          1912
   Ala Gln Glu Ala Ala Gln Gln Ala Gln Ala Ala Glu Glu Ala Gln Ala
   315                 320                 325                 330
   gcg caa gca gct gag aca gca caa gcc caa gcc gcg caa gct gcg gaa          1960
   Ala Gln Ala Ala Glu Thr Ala Gln Ala Gln Ala Ala Gln Ala Ala Glu
                   335                 340                 345
   acc caa gct gca caa gcc gcg caa gct cag gca gaa gcg aat gat cgt          2008
   Thr Gln Ala Ala Gln Ala Ala Gln Ala Gln Ala Glu Ala Asn Asp Arg
               350                 355                 360
   gcc gcc gcg caa cag cgt gct gca gag gct caa gca gca gcg gaa cag          2056
   Ala Ala Ala Gln Gln Arg Ala Ala Glu Ala Gln Ala Ala Ala Glu Gln
           365                 370                 375
   gcg caa cgt gag gct gac gct cag gcg gcc aac gat gcc caa gct cag          2104
   Ala Gln Arg Glu Ala Asp Ala Gln Ala Ala Asn Asp Ala Gln Ala Gln
       380                 385                 390
   gca ctg cgt gaa cag gcg ctc acc gca gcc tcc atc gct gcg gct gct          2152
   Ala Leu Arg Glu Gln Ala Leu Thr Ala Ala Ser Ile Ala Ala Ala Ala
   395                 400                 405                 410
   cta att gcg gcg agc cag tcc agc cat gcc act act caa aat cct tac       2200
   Leu Ile Ala Ala Ser Gln Ser Ser His Ala Thr Thr Gln Asn Pro Tyr
                   415                 420                 425
   cca act gat gaa gac gcg gat ccg acc gat att gcg gac atc caa ggc       2248
   Pro Thr Asp Glu Asp Ala Asp Pro Thr Asp Ile Ala Asp Ile Gln Gly
               430                 435                 440
   cca acg cag cca ggt acg gat gag tct gga gat ttc cag agc aac tcc       2296
   Pro Thr Gln Pro Gly Thr Asp Glu Ser Gly Asp Phe Gln Ser Asn Ser
           445                 450                 455
   agc gac aac gat tcc aca ggc aac gat tcc aca ggc tct gac tct tca       2344
   Ser Asp Asn Asp Ser Thr Gly Asn Asp Ser Thr Gly Ser Asp Ser Ser
       460                 465                 470
   gat tca gat tcc tcc ggc aac gat tct tca gag gtt att tcc ggc gat       2392
   Asp Ser Asp Ser Ser Gly Asn Asp Ser Ser Glu Val Ile Ser Gly Asp
   475                 480                 485                 490
   cgt tcc gct cag att gag act gtg att gcg cgc gcc atg agc cag ttg       2440
   Arg Ser Ala Gln Ile Glu Thr Val Ile Ala Arg Ala Met Ser Gln Leu
                   495                 500                 505
   ggt gtg cag tac gca tgg ggt ggc ggt aac gct aat ggc cca act ctg       2488
   Gly Val Gln Tyr Ala Trp Gly Gly Gly Asn Ala Asn Gly Pro Thr Leu
               510                 515                 520
   ggt atc cgt gac ggt ggc gtg gcg gac tct tac ggc gat tac aac aag       2536
   Gly Ile Arg Asp Gly Gly Val Ala Asp Ser Tyr Gly Asp Tyr Asn Lys
           525                 530                 535
   gtt ggc ttc gac tgc tct gga ctg acc ttg tat gcg ttt gcg ggt gtg       2584
   Val Gly Phe Asp Cys Ser Gly Leu Thr Leu Tyr Ala Phe Ala Gly Val
       540                 545                 550
   gga att tca ctt cct cac tac acg ggc tac cag tac cag cac ggc acc       2632
   Gly Ile Ser Leu Pro His Tyr Thr Gly Tyr Gln Tyr Gln His Gly Thr
   555                 560                 565                 570
   aag gtg tcg cct tct gag atg caa cgt ggc gat ctg atc ttc tat ggt       2680
   Lys Val Ser Pro Ser Glu Met Gln Arg Gly Asp Leu Ile Phe Tyr Gly
                   575                 580                 585
   ccg gga gcg tct cag cac gtg gca att tac ctc ggt gat ggt cag atg       2728
   Pro Gly Ala Ser Gln His Val Ala Ile Tyr Leu Gly Asp Gly Gln Met
               590                 595                 600
   att gag gct ccg aat tcg ggt tct gtc gtg aag att tct cct gtt cgc       2776
   Ile Glu Ala Pro Asn Ser Gly Ser Val Val Lys Ile Ser Pro Val Arg
           605                 610                 615
   tgg agc gga atg acc gag agc gtg gta cgc ctc att tagtttcctc            2822
   Trp Ser Gly Met Thr Glu Ser Val Val Arg Leu Ile
       620                 625                 630
   ctatgaatct tgatgtggtt catgcgtttt tatgcaatat caaccaaaag ttggtacgat     2882
   cctcatatga atgaacgcac atcggatgca tttgacgccc tccttgtgct ctccttcggt     2942
   ggtcccgaag ggcacgagga ggttcgtccg tttttggaga atgtcactca cggaaggggg     3002
   attccgccgg aacgtctaga tgaagtggcg gttcattacc accacttcgg tggtatcagc 3062
   cccatcaatg cgctgaacag ggaaattatc gccaatgtgg aaaaagaatt ggcgtctcgc 3122
   gatcacaagc tgcctgttta ttttggtaac cgcaactgga agccgtttga taatgaggcc 3182
   gctgaacaaa tggctgatga cggcgtgaaa aacgcgctgg tgttggcaac ttccgcttgg 3242
   ggtggctact ccggttgtcg gcagtaccag gaagatattc agggcatgat caagcacctg 3302
   gagtctcagg ggcagtcgat cacgttcacc aagctgcgtc agttctacga tcaccctcgt 3362
   tttgtctcca ccatggctca attggttcag gattcctacg cgaagcttcc cgatgagctg 3422
   cgagatgagg cgcgtctggt cttcaccgcg cactccattc cactgactgc ggacaatgct 3482
   gcgggaaccc ctgaggatgg ctccttgtat tccacacagg tcaaggaagc gtcagcactg 3542
   attgctgagg ctgttggtgt gtcagatttt gatgtggtgt ggcagtcccg ctcgggtagc 3602
   ccgcacactc cgtggctgga gcctgacatc gtggatcacg cagtggagct caacgagaag 3662
   ggtcaaaaag cgctcgttgt ctgccctgta ggctttattt ctgatcatat ggaagtcatt 3722
   tgggatcttg attccgagct gatggaagaa gccgagaagc gcaacatggt ggtcgagcgt 3782
   gtcgctaccg ttggccccac cgatgaattc gca 3815
<210> 4
   <211> 630
   <212> PRT
   <213> Corynebacterium glutamicum
<400> 4
   Met Ala Asn Leu Asp Ser Val Arg Pro Gly Val Arg Ala Ala Leu Arg
   1               5                   10                  15
   Arg Thr His Thr Gln Val Lys Gly Val Thr Leu Thr Arg Ala Leu Ile
               20                  25                  30
   Ala Leu Ala Val Ser Gly Ala Leu Leu Ser Ser Met Thr Pro Ala Val
           35                  40                  45
   Ala Gln Pro Gln Asn Pro Asp Asp Ala Ala Ile Ala Gln Ala Glu Glu
       50                  55                  60
   Asn Val Ser Ala Gly Asp Gly Glu Val Ala Arg Leu Ala Gly Ser Leu
   65                  70                  75                  80
   Ser Ser Thr Asp Ala Glu Ile Asn Arg Ile Glu Leu Glu Met Gly Ala
                   85                  90                  95
   Leu Arg Glu Glu Val Asn Lys Ser Leu Val Asp Leu His Asp Ala Gln
               100                 105                 110
   Ala Ile Ala Glu Gln Ala Arg Gln Asp Ala Leu Ala Ala Lys Lys Asp
   115 120 125
   Leu Asp Asp Ser Gln Ala Gln Ile Glu Ala Ala Gln Glu Arg Leu Asp
   130 135 140
   Glu Ile Ser Arg Ala Ala Tyr Arg Gln Asn Gly Thr Ser Lys Arg Leu
   145 150 155 160
   Ser Gly Ile Ser Gly Asn Gly Asn Ser Glu Asp Ala Leu Asp Arg Gln
   165 170 175
   Thr Tyr Leu Arg Thr Ser Ala Glu Lys Gln Gln Ala Ala Val Glu Glu
   180 185 190
   Leu Asp Arg Leu Arg Thr Glu Asn Ala Asn Lys Glu Ser Val Leu Arg
   195 200 205
   Gln Ala Arg Ile Val Ala Glu Gln Arg Glu Ala Glu Ala Val Glu Lys
   210 215 220
   Gln Val Gln Thr Glu Ala Ala Ile Ala Ala Asn Ser Glu Gln Leu Asn
   225 230 235 240
   Val Leu Thr Asn Asn Arg Ser Thr Leu Val Ala Gln Arg Asp Gly Ala
   245 250 255
   Glu Arg Asn Leu Ala Ile Ala Arg Ala Gln Ala Asp Asn Leu Gln Gly
   260 265 270
   Gln Arg Ala Glu Tyr Glu Glu Phe Gln Gln Ala Glu Gln Ala Arg Ile
   275 280 285
   Gln Ala Glu Ala Glu Ala Gln Ala Ala Ala Glu Glu Lys Arg Arg Ala
   290 295 300
   Asp Glu Ala Ala Ala Gln Ala Ala Ala Glu Ala Gln Glu Ala Ala Gln
   305 310 315 320
   Gln Ala Gln Ala Ala Glu Glu Ala Gln Ala Ala Gln Ala Ala Glu Thr
   325 330 335
   Ala Gln Ala Gln Ala Ala Gln Ala Ala Glu Thr Gln Ala Ala Gln Ala
   340 345 350
   Ala Gln Ala Gln Ala Glu Ala Asn Asp Arg Ala Ala Ala Gln Gln Arg
           355                 360                 365
   Ala Ala Glu Ala Gln Ala Ala Ala Glu Gln Ala Gln Arg Glu Ala Asp
       370                 375                 380
   Ala Gln Ala Ala Asn Asp Ala Gln Ala Gln Ala Leu Arg Glu Gln Ala
   385                 390                 395                 400
   Leu Thr Ala Ala Ser Ile Ala Ala Ala Ala Leu Ile Ala Ala Ser Gln
                   405                 410                 415
   Ser Ser His Ala Thr Thr Gln Asn Pro Tyr Pro Thr Asp Glu Asp Ala
               420                 425                 430
   Asp Pro Thr Asp Ile Ala Asp Ile Gln Gly Pro Thr Gln Pro Gly Thr
           435                 440                 445
   Asp Glu Ser Gly Asp Phe Gln Ser Asn Ser Ser Asp Asn Asp Ser Thr
       450                 455                 460
   Gly Asn Asp Ser Thr Gly Ser Asp Ser Ser Asp Ser Asp Ser Ser Gly
   465                 470                 475                 480
   Asn Asp Ser Ser Glu Val Ile Ser Gly Asp Arg Ser Ala Gln Ile Glu
                   485                 490                 495
   Thr Val Ile Ala Arg Ala Met Ser Gln Leu Gly Val Gln Tyr Ala Trp
               500                 505                 510
   Gly Gly Gly Asn Ala Asn Gly Pro Thr Leu Gly Ile Arg Asp Gly Gly
           515                 520                 525
   Val Ala Asp Ser Tyr Gly Asp Tyr Asn Lys Val Gly Phe Asp Cys Ser
       530                 535                 540
   Gly Leu Thr Leu Tyr Ala Phe Ala Gly Val Gly Ile Ser Leu Pro His
   545                 550                 555                 560
   Tyr Thr Gly Tyr Gln Tyr Gln His Gly Thr Lys Val Ser Pro Ser Glu
                   565                 570                 575
   Met Gln Arg Gly Asp Leu Ile Phe Tyr Gly Pro Gly Ala Ser Gln His
               580                 585                 590
   Val Ala Ile Tyr Leu Gly Asp Gly Gln Met Ile Glu Ala Pro Asn Ser
           595                 600                 605
   Gly Ser Val Val Lys Ile Ser Pro Val Arg Trp Ser Gly Met Thr Glu
       610                 615                 620
   Ser Val Val Arg Leu Ile
   625                 630
<210> 5
   <211> 1266
   <212> DNA
   <213> Corynebacterium glutamicum ATCC13032
<220>
   <221> CDS
   <222> (1)..(1263)
   <223> cds of aspartokinase gene
<220>
   <221> misc_feature
   <222> (1264)..(1266)
<400> 5
   gtg gcc ctg gtc gta cag aaa tat ggc ggt tcc tcg ctt gag agt gcg          48
   Met Ala Leu Val Val Gln Lys Tyr Gly Gly Ser Ser Leu Glu Ser Ala
   1               5                   10                  15
   gaa cgc att aga aac gtc gct gaa cgg atc gtt gcc acc aag aag gct          96
   Glu Arg Ile Arg Asn Val Ala Glu Arg Ile Val Ala Thr Lys Lys Ala
               20                  25                  30
   gga aat gat gtc gtg gtt gtc tgc tcc gca atg gga gac acc acg gat         144
   Gly Asn Asp Val Val Val Val Cys Ser Ala Met Gly Asp Thr Thr Asp
           35                  40                  45
   gaa ctt cta gaa ctt gca gcg gca gtg aat ccc gtt ccg cca gct cgt         192
   Glu Leu Leu Glu Leu Ala Ala Ala Val Asn Pro Val Pro Pro Ala Arg
       50                  55                  60
   gaa atg gat atg ctc ctg act gct ggt gag cgt att tct aac gct ctc         240
   Glu Met Asp Met Leu Leu Thr Ala Gly Glu Arg Ile Ser Asn Ala Leu
   65                  70                  75                  80
   gtc gcc atg gct att gag tcc ctt ggc gca gaa gcc caa tct ttc acg         288
   Val Ala Met Ala Ile Glu Ser Leu Gly Ala Glu Ala Gln Ser Phe Thr
                   85                  90                  95
   ggc tct cag gct ggt gtg ctc acc acc gag cgc cac gga aac gca cgc         336
   Gly Ser Gln Ala Gly Val Leu Thr Thr Glu Arg His Gly Asn Ala Arg
               100                 105                 110
   att gtt gat gtc act cca ggt cgt gtg cgt gaa gca ctc gat gag ggc         384
   Ile Val Asp Val Thr Pro Gly Arg Val Arg Glu Ala Leu Asp Glu Gly
           115                 120                 125
   aag atc tgc att gtt gct ggt ttc cag ggt gtt aat aaa gaa acc cgc         432
   Lys Ile Cys Ile Val Ala Gly Phe Gln Gly Val Asn Lys Glu Thr Arg
       130                 135                 140
   gat gtc acc acg ttg ggt cgt ggt ggt tct gac acc act gca gtt gcg         480
   Asp Val Thr Thr Leu Gly Arg Gly Gly Ser Asp Thr Thr Ala Val Ala
   145                 150                 155                 160
   ttg gca gct gct ttg aac gct gat gtg tgt gag att tac tcg gac gtt        528
   Leu Ala Ala Ala Leu Asn Ala Asp Val Cys Glu Ile Tyr Ser Asp Val
                   165                 170                 175
   gac ggt gtg tat acc gct gac ccg cgc atc gtt cct aat gca cag aag        576
   Asp Gly Val Tyr Thr Ala Asp Pro Arg Ile Val Pro Asn Ala Gln Lys
               180                 185                 190
   ctg gaa aag ctc agc ttc gaa gaa atg ctg gaa ctt gct gct gtt ggc        624
   Leu Glu Lys Leu Ser Phe Glu Glu Met Leu Glu Leu Ala Ala Val Gly
           195                 200                 205
   tcc aag att ttg gtg ctg cgc agt gtt gaa tac gct cgt gca ttc aat        672
   Ser Lys Ile Leu Val Leu Arg Ser Val Glu Tyr Ala Arg Ala Phe Asn
       210                 215                 220
   gtg cca ctt cgc gta cgc tcg tct tat agt aat gat ccc ggc act ttg        720
   Val Pro Leu Arg Val Arg Ser Ser Tyr Ser Asn Asp Pro Gly Thr Leu
   225                 230                 235                 240
   att gcc ggc tct atg gag gat att cct gtg gaa gaa gca gtc ctt acc        768
   Ile Ala Gly Ser Met Glu Asp Ile Pro Val Glu Glu Ala Val Leu Thr
                   245                 250                 255
   ggt gtc gca acc gac aag tcc gaa gcc aaa gta acc gtt ctg ggt att        816
   Gly Val Ala Thr Asp Lys Ser Glu Ala Lys Val Thr Val Leu Gly Ile
               260                 265                 270
   tcc gat aag cca ggc gag gct gcg aag gtt ttc cgt gcg ttg gct gat        864
   Ser Asp Lys Pro Gly Glu Ala Ala Lys Val Phe Arg Ala Leu Ala Asp
           275                 280                 285
   gca gaa atc aac att gac atg gtt ctg cag aac gtc tct tct gta gaa        912
   Ala Glu Ile Asn Ile Asp Met Val Leu Gln Asn Val Ser Ser Val Glu
       290                 295                 300
   gac ggc acc acc gac atc acc ttc acc tgc cct cgt tcc gac ggc cgc        960
   Asp Gly Thr Thr Asp Ile Thr Phe Thr Cys Pro Arg Ser Asp Gly Arg
   305                 310                 315                 320
   cgc gcg atg gag atc ttg aag aag ctt cag gtt cag ggc aac tgg acc       1008
   Arg Ala Met Glu Ile Leu Lys Lys Leu Gln Val Gln Gly Asn Trp Thr
                   325                 330                 335
   aat gtg ctt tac gac gac cag gtc ggc aaa gtc tcc ctc gtg ggt gct       1056
   Asn Val Leu Tyr Asp Asp Gln Val Gly Lys Val Ser Leu Val Gly Ala
               340                 345                 350
   ggc atg aag tct cac cca ggt gtt acc gca gag ttc atg gaa gct ctg       1104
   Gly Met Lys Ser His Pro Gly Val Thr Ala Glu Phe Met Glu Ala Leu
           355                 360                 365
   cgc gat gtc aac gtg aac atc gaa ttg att tcc acc tct gag att cgt       1152
   Arg Asp Val Asn Val Asn Ile Glu Leu Ile Ser Thr Ser Glu Ile Arg
       370                 375                 380
   att tcc gtg ctg atc cgt gaa gat gat ctg gat gct gct gca cgt gca       1200
   Ile Ser Val Leu Ile Arg Glu Asp Asp Leu Asp Ala Ala Ala Arg Ala
   385                 390                 395                 400
   ttg cat gag cag ttc cag ctg ggc ggc gaa gac gaa gcc gtc gtt tat       1248
   Leu His Glu Gln Phe Gln Leu Gly Gly Glu Asp Glu Ala Val Val Tyr
   405 410 415
   gca ggc acc gga cgc taa 1266
   Ala Gly Thr Gly Arg
   420
<210> 6
   <211> 421
   <212> PRT
   <213> Corynebacterium glutamicum ATCC13032
<400> 6
   Met Ala Leu Val Val Gln Lys Tyr Gly Gly Ser Ser Leu Glu Ser Ala
   1               5                   10                  15
   Glu Arg Ile Arg Asn Val Ala Glu Arg Ile Val Ala Thr Lys Lys Ala
               20                  25                  30
   Gly Asn Asp Val Val Val Val Cys Ser Ala Met Gly Asp Thr Thr Asp
           35                  40                  45
   Glu Leu Leu Glu Leu Ala Ala Ala Val Asn Pro Val Pro Pro Ala Arg
       50                  55                  60
   Glu Met Asp Met Leu Leu Thr Ala Gly Glu Arg Ile Ser Asn Ala Leu
   65                  70                  75                  80
   Val Ala Met Ala Ile Glu Ser Leu Gly Ala Glu Ala Gln Ser Phe Thr
                   85                  90                  95
   Gly Ser Gln Ala Gly Val Leu Thr Thr Glu Arg His Gly Asn Ala Arg
               100                 105                 110
   Ile Val Asp Val Thr Pro Gly Arg Val Arg Glu Ala Leu Asp Glu Gly
           115                 120                 125
   Lys Ile Cys Ile Val Ala Gly Phe Gln Gly Val Asn Lys Glu Thr Arg
       130                 135                 140
   Asp Val Thr Thr Leu Gly Arg Gly Gly Ser Asp Thr Thr Ala Val Ala
   145                 150                 155                 160
   Leu Ala Ala Ala Leu Asn Ala Asp Val Cys Glu Ile Tyr Ser Asp Val
                   165                 170                 175
   Asp Gly Val Tyr Thr Ala Asp Pro Arg Ile Val Pro Asn Ala Gln Lys
               180                 185                 190
   Leu Glu Lys Leu Ser Phe Glu Glu Met Leu Glu Leu Ala Ala Val Gly
           195                 200                 205
   Ser Lys Ile Leu Val Leu Arg Ser Val Glu Tyr Ala Arg Ala Phe Asn
       210                 215                 220
   Val Pro Leu Arg Val Arg Ser Ser Tyr Ser Asn Asp Pro Gly Thr Leu
   225                 230                 235                 240
   Ile Ala Gly Ser Met Glu Asp Ile Pro Val Glu Glu Ala Val Leu Thr
                   245                 250                 255
   Gly Val Ala Thr Asp Lys Ser Glu Ala Lys Val Thr Val Leu Gly Ile
               260                 265                 270
   Ser Asp Lys Pro Gly Glu Ala Ala Lys Val Phe Arg Ala Leu Ala Asp
           275                 280                 285
   Ala Glu Ile Asn Ile Asp Met Val Leu Gln Asn Val Ser Ser Val Glu
       290                 295                 300
   Asp Gly Thr Thr Asp Ile Thr Phe Thr Cys Pro Arg Ser Asp Gly Arg
   305                 310                 315                 320
   Arg Ala Met Glu Ile Leu Lys Lys Leu Gln Val Gln Gly Asn Trp Thr
                   325                 330                 335
   Asn Val Leu Tyr Asp Asp Gln Val Gly Lys Val Ser Leu Val Gly Ala
               340                 345                 350
   Gly Met Lys Ser His Pro Gly Val Thr Ala Glu Phe Met Glu Ala Leu
           355                 360                 365
   Arg Asp Val Asn Val Asn Ile Glu Leu Ile Ser Thr Ser Glu Ile Arg
       370                 375                 380
   Ile Ser Val Leu Ile Arg Glu Asp Asp Leu Asp Ala Ala Ala Arg Ala
   385                 390                 395                 400
   Leu His Glu Gln Phe Gln Leu Gly Gly Glu Asp Glu Ala Val Val Tyr
                   405                 410                 415
   Ala Gly Thr Gly Arg
               420
<210> 7
   <211> 1245
   <212> DNA
   <213> Corynebacterium glutamicum ATCC13032
<220>
   <221> CDS
   <222> (1)..(1242)
   <223> coding sequence of NCgl2986
<220>
   <221> misc_feature
   <222> (1243)..(1245)
   <223> taa stop codon <400> 7
<210> 8
   <211> 414
   <212> PRT
   <213> Corynebacterium glutamicum ATCC13032
<400> 8
   Met Asp Glu Leu Tyr Pro Leu Ile Ile Leu Asn Met Asn Asp Gly Arg
   1               5                   10                  15
   Ser Arg Val Ser Lys Val Leu Arg Val Gly Asp Arg Ser Pro Arg Val
               20                  25                  30
   Ala Glu Val Arg Thr Thr Leu Ala Arg Leu Gly Val Ile Glu Gly Tyr
           35                  40                  45
   Ser Arg Glu Met Ser Ala Lys Thr Glu Ser Gln Lys Phe His Glu Glu
       50                  55                  60
   Glu Thr Leu Phe Asp Glu Glu Leu Ser Leu Ser Ile Lys Ser Phe Gln
   65                  70                  75                  80
   Gln Ala Arg Gly Val Val Pro Ser Gly Leu Ile Asp Asp Pro Thr Leu
                   85                  90                  95
   Arg Ala Ile Arg Glu Ala Ser Tyr Thr Leu Gly Thr Arg Val Leu Ala
               100                 105                 110
   Tyr Gln Pro Gly Asn Gln Leu Val Gly Asp Asp Val Val Glu Ile Gln
           115                 120                 125
   Ser His Leu Gln Glu Leu Gly Phe Tyr Ala Asp Arg Val Asp Gly His
       130                 135                 140
   Phe Gly Glu Leu Thr His Lys Ala Val Met Asn Tyr Gln Leu Asn Tyr
   145                 150                 155                 160
   Gly Met Gln Val Asp Gly Ile Cys Gly Pro Asp Thr Ile Arg Ala Leu
                   165                 170                 175
   Ser Arg Leu Gly Leu Arg Ile Lys Gly Gly Ser Ala Gln Ala Ile Arg
               180                 185                 190
   Glu Arg Glu Arg Met Arg Asn Ala Gly Pro Arg Leu Ala Gly Lys Arg
           195                 200                 205
   Val Val Ile Asp Pro Ala Leu Gly Gly Ser Asn Lys Gly Gln Ile Val
       210                 215                 220
   Lys Gly Pro Tyr Gly Glu Ile Ser Glu Glu Glu Ile Leu Trp Asp Leu
   225                 230                 235                 240
   Ala Thr Arg Leu Glu Gly Arg Met Ile Ala Thr Gly Met Glu Thr Ile
                   245                 250                 255
   Leu Ser Arg Pro His Met Asp Asp Pro Ser Ser Arg Asp Arg Ala Ser
   260 265 270
   Ile Ala Asn Ala Phe Gly Ala Asp Leu Met Leu Ser Leu His Cys Asp
   275 280 285
   Ser Tyr Pro Asn Glu Lys Ala Asn Gly Val Ala Ser Phe Tyr Phe Gly
   290 295 300
   Ser Glu Asn Gly Thr Asn Ser Leu Thr Gly Glu Thr Leu Ser Ala Tyr
   305 310 315 320
   Ile Gln Lys Glu Ile Val Ala Arg Thr Pro Leu Asn Asn Cys Gly Ser
   325 330 335
   His Ala Arg Thr Trp Asp Leu Leu Arg Leu Thr Arg Met Pro Met Val
   340 345 350
   Glu Val Val Thr Gly Tyr Leu Thr Asn Pro Asp Asp Leu Ala Val Leu
   355 360 365
   Thr Asp Pro Gln Met Arg Asp His Ile Ala Glu Ala Ile Val Val Ala
   370 375 380
   Val Lys Arg Leu Tyr Leu Leu Asp Glu Glu Ala Gln Pro Lys Thr Gly
   385 390 395 400
   Thr Phe Lys Phe Ser Glu Leu Leu Gln Ser Glu Gln Ala Gly
   405 410
<210> 9
   <211> 180
   <212> PRT
   <213> Corynebacterium glutamicum
<220>
   <221> MISC_FEATURE
   <222> (1)..(180)
   <223> N terminus of the polypeptide identified by locus_tag cgR_1596
<400> 9
   Met Ala Asn Leu Asp Ser Val Arg Pro Gly Val Arg Ala Ala Leu Arg
   1               5                   10                  15
   Arg Thr His Thr Gln Val Lys Gly Val Ala Leu Thr Arg Ala Leu Ile
               20                  25                  30
   Ala Leu Ala Val Ser Gly Ala Leu Leu Ser Ser Met Thr Pro Ala Val
           35                  40                  45
   Ala Gln Pro Gln Asn Pro Asp Asp Ala Ala Ile Ala Gln Ala Glu Glu
       50                  55                  60
   Asn Val Ser Ala Gly Asp Gly Glu Val Ala Arg Leu Ala Gly Ser Leu
   65                  70                  75                  80
   Ser Ser Thr Asp Ala Glu Ile Asn Arg Val Glu Leu Glu Met Gly Ala
                   85                  90                  95
   Leu Arg Glu Glu Val Asn Lys Ser Leu Val Asp Leu His Asp Ala Gln
               100                 105                 110
   Ala Ile Ala Glu Gln Ala Arg Gln Asp Ala Leu Ala Ala Lys Lys Asp
           115                 120                 125
   Leu Asp Asp Ser Gln Ala Gln Ile Glu Ala Ala Gln Glu Arg Leu Asp
       130                 135                 140
   Glu Ile Ser Arg Ala Ala Tyr Arg Gln Asn Gly Thr Ser Lys Gly Leu
   145                 150                 155                 160
   Ser Gly Ile Ser Gly Asn Gly Asn Ser Glu Asp Ala Leu Asp Arg Gln
                   165                 170                 175
   Thr Tyr Leu Arg
               180
<210> 10
   <211> 154
   <212> PRT
   <213> Corynebacterium glutamicum
<220>
   <221> MISC_FEATURE
   <222> (1)..(154)
   <223> N terminus of the polypeptide identified by locus_tag NCgl1480
<400> 10
   Met Thr Arg Ala Leu Ile Ala Leu Ala Val Ser Gly Ala Leu Leu Ser
   1               5                   10                  15
   Ser Met Thr Pro Ala Val Ala Gln Pro Gln Asn Pro Asp Asp Ala Ala
               20                  25                  30
   Ile Ala Gln Ala Glu Glu Asn Val Ser Ala Gly Asp Gly Glu Val Ala
           35                  40                  45
   Arg Leu Ala Gly Ser Leu Ser Ser Thr Asp Ala Glu Ile Asn Arg Val
       50                  55                  60
   Glu Leu Glu Met Gly Ala Leu Arg Glu Glu Val Asn Lys Ser Leu Val
   65                  70                  75                  80
   Asp Leu His Asp Ala Gln Ala Ile Ala Glu Gln Ala Arg Gln Asp Ala
                   85                  90                  95
   Leu Ala Ala Lys Lys Asp Leu Asp Asp Ser Gln Ala Gln Ile Glu Ala
               100                 105                 110
   Ala Gln Glu Arg Leu Asp Glu Ile Ser Arg Ala Ala Tyr Arg Gln Asn
           115                 120                 125
   Gly Thr Ser Lys Gly Leu Ser Gly Ile Ser Gly Asn Gly Asn Ser Glu
       130                 135                 140
   Asp Ala Leu Asp Arg Gln Thr Tyr Leu Arg
   145                 150
<210> 11
   <211> 2715
   <212> DNA
   <213> artificial sequence
<220>
   <223> polynucleotide NCgl1480*_Mut
<220>
   <221> misc_feature
   <222> (4)..(11)
   <223> recognition site for restriction endonuclease SbfI
<220>
   <221> misc_feature
   <222> (2705)..(2712)
   <223> recognition site for restriction endonuclease SbfI
<400> 11
   gatcctgcag gcgtcgaaaa gcaatatctt tataagtaaa gttctaaagc tttacttata      60
   gcactttcgg taggtgtcga gaaaattccc aaattgcgga aaattcacat actttgtgcc     120
   acaaagtgat aaacatcaca aaatttcatg actcagccat tcatgcaggc cagaacatgc     180
   aaaaccttgc acgctctggc cttttgttat gtaactgttg tgaaagatgt atcaaaagtt     240
   accaaggtga cttaatgttc agatatcgac tcatgggttg ttttccaaaa cttgaatcaa     300
   tttgagtaac agtagttatc aagcgttaaa ccttgaaact tccactcttt ttactgatgg     360
   gcatttgggc aaacggggaa gcttgctgga gatgcaataa atcggtgaat ggttggtgca     420
   atgaagagga gagtggccca tagtttccgc tacggaacgc ggagtcttta cgtttcaatt     480
   cttggcaagt atctagcgtc aaggcttgat atgtgaaggg attcggattg aacaggagaa 540
   catgtggcca atctagattc agttcgccct ggggtgcggg cagctcttcg acgtacccat 600
   acccaggtaa aaggtgtcac gttgaccagg gcgttgattg cgcttgcagt aagcggagct 660
   ttgcttagtt ccatgactcc ggcggtggcg cagccacaga atccggatga cgcagccatt 720
   gcacaggcag aggaaaatgt ttcggcgggc gatggggaag tcgcccgcct ggcaggatct 780
   ttgtccagca ctgacgcgga aattaaccgc atcgagctgg aaatgggtgc tctgcgtgaa 840
   gaagtgaaca agtccctcgt ggatttgcat gatgcgcagg caatcgccga gcaggcccgc 900
   caagatgcac ttgcagccaa gaaggatctc gatgattctc aagcgcagat cgaagcagcc 960
   caagagcgcc ttgatgagat ttcacgtgca gcgtatcgcc aaaacggaac ctccaagagg 1020
   ctttcaggca tctcgggcaa tggaaattct gaagatgcgc tagatcgtca gacttacctg 1080
   cgaaccagtg cggaaaagca gcaggcagct gttgaagagc ttgatcgcct ccgtacggaa 1140
   aacgccaaca aggaatcggt gttgcgccag gcccgcatcg ttgctgagca gcgtgaggcg 1200
   gaagccgtcg aaaagcaagt ccagaccgag gctgcaattg ccgcaaacag cgagcagctc 1260
   aatgtcttga ctaacaatcg cagtaccttg gttgcccagc gtgatggggc tgagcgcaac 1320
   ttggccatcg ctcgtgcgca ggcggataat ctgcaaggtc agcgtgctga gtacgaggaa 1380
   ttccagcagg cagagcaggc tcgcatccag gcggaagcgg aagctcaggc tgctgcggag 1440
   gagaagcgtc gtgccgatga ggctgctgca caggcagccg ctgaagctca agaagctgcc 1500
   cagcaagctc aggcggcgga ggaagcccaa gccgcgcaag cagctgagac agcacaagcc 1560
   caagccgcgc aagctgcgga aacccaagct gcacaagccg cgcaagctca ggcagaagcg 1620
   aatgatcgtg ccgccgcgca acagcgtgct gcagaggctc aagcagcagc ggaacaggcg 1680
   caacgtgagg ctgacgctca ggcggccaac gatgcccaag ctcaggcact gcgtgaacag 1740
   gcgctcaccg cagcctccat cgctgcggct gctctaattg cggcgagcca gtccagccat 1800
   gccactactc aaaatcctta cccaactgat gaagacgcgg atccgaccga tattgcggac 1860
   atccaaggcc caacgcagcc aggtacggat gagtctggag atttccagag caactccagc 1920
   gacaacgatt ccacaggcaa cgattccaca ggctctgact cttcagattc agattcctcc 1980
   ggcaacgatt cttcagaggt tatttccggc gatcgttccg ctcagattga gactgtgatt 2040
   gcgcgcgcca tgagccagtt gggtgtgcag tacgcatggg gtggcggtaa cgctaatggc 2100
   ccaactctgg gtatccgtga cggtggcgtg gcggactctt acggcgatta caacaaggtt 2160
   ggcttcgact gctctggact gaccttgtat gcgtttgcgg gtgtgggaat ttcacttcct 2220
   cactacacgg gctaccagta ccagcacggc accaaggtgt cgccttctga gatgcaacgt 2280
   ggcgatctga tcttctatgg tccgggagcg tctcagcacg tggcaattta cctcggtgat 2340
   ggtcagatga ttgaggctcc gaattcgggt tctgtcgtga agatttctcc tgttcgctgg     2400
   agcggaatga ccgagagcgt ggtacgcctc atttagtttc ctcctatgaa tcttgatgtg     2460
   gttcatgcgt ttttatgcaa tatcaaccaa aagttggtac gatcctcata tgaatgaacg     2520
   cacatcggat gcatttgacg ccctccttgt gctctccttc ggtggtcccg aagggcacga     2580
   ggaggttcgt ccgtttttgg agaatgtcac tcacggaagg gggattccgc cggaacgtct     2640
   agatgaagtg gcggttcatt accaccactt cggtggtatc agccccatca atgcgctgaa     2700
   caggcctgca ggatc                                                      2715
<210> 12
   <211> 20
   <212> DNA
   <213> artificial sequence
<220>
   <223> primer LC-NCgl1480_1
<400> 12
   agcggagctt tgcttagttc 20
<210> 13
   <211> 20
   <212> DNA
   <213> artificial sequence
<220>
   <223> primer LC-NCgl1480_2
<400> 13
   tgcttcgatc tgcgcttgag 20
<210> 14
   <211> 20
   <212> DNA
   <213> artificial sequence
<220>
   <223> probe NCgl1480_90_C
<400> 14
   ccagctcgat gcggttaatt 20
<210> 15
   <211> 35
   <212> DNA
   <213> artificial sequence
<220>
   <223> probe NCgl1480_90_A
<400> 15
   agggacttgt tcacttcttc acgcagagca cccat 35
<210> 16
   <211> 20
   <212> DNA
   <213> artificial sequence
<220>
   <223> primer LC-NCgl1480_3
<400> 16
   cgctgcggct gctctaattg 20
<210> 17
   <211> 20
   <212> DNA
   <213> artificial sequence
<220>
   <223> primer LC-NCgl1480_4
<400> 17
   gcgtactgca cacccaactg 20
<210> 18
   <211> 20
   <212> DNA
   <213> artificial sequence
<220>
   <223> probe NCgl1480_454_C
<400> 18
   gttgctctgg aaatctccag 20
<210> 19
   <211> 35
   <212> DNA
   <213> artificial sequence
<220>
   <223> probe NCgl1480_454_A
<400> 19
   ctgtggaatc gttgcctgtg gaatcgttgt cgctg 35
<210> 20
   <211> 1326
   <212> DNA
   <213> Corynebacterium glutamicum
<400> 20
   atccggatga cgcagccatt gcacaggcag aggaaaatgt ttcggcgggc gatggggaag      60
   tcgcccgcct ggcaggatct ttgtccagca ctgacgcgga aattaaccgc atcgagctgg     120
   aaatgggtgc tctgcgtgaa gaagtgaaca agtccctcgt ggatttgcat gatgcgcagg     180
   caatcgccga gcaggcccgc caagatgcac ttgcagccaa gaaggatctc gatgattctc     240
   aagcgcagat cgaagcagcc caagagcgcc ttgatgagat ttcacgtgca gcgtatcgcc     300
   aaaacggaac ctccaagagg ctttcaggca tctcgggcaa tggaaattct gaagatgcgc     360
   tagatcgtca gacttacctg cgaaccagtg cggaaaagca gcaggcagct gttgaagagc     420
   ttgatcgcct ccgtacggaa aacgccaaca aggaatcggt gttgcgccag gcccgcatcg     480
   ttgctgagca gcgtgaggcg gaagccgtcg aaaagcaagt ccagaccgag gctgcaattg     540
   ccgcaaacag cgagcagctc aatgtcttga ctaacaatcg cagtaccttg gttgcccagc     600
   gtgatggggc tgagcgcaac ttggccatcg ctcgtgcgca ggcggataat ctgcaaggtc     660
   agcgtgctga gtacgaggaa ttccagcagg cagagcaggc tcgcatccag gcggaagcgg     720
   aagctcaggc tgctgcggag gagaagcgtc gtgccgatga ggctgctgca caggcagccg     780
   ctgaagctca agaagctgcc cagcaagctc aggcggcgga ggaagcccaa gccgcgcaag     840
   cagctgagac agcacaagcc caagccgcgc aagctgcgga aacccaagct gcacaagccg     900
   cgcaagctca ggcagaagcg aatgatcgtg ccgccgcgca acagcgtgct gcagaggctc     960
   aagcagcagc ggaacaggcg caacgtgagg ctgacgctca ggcggccaac gatgcccaag    1020
   ctcaggcact gcgtgaacag gcgctcaccg cagcctccat cgctgcggct gctctaattg    1080
   cggcgagcca gtccagccat gccactactc aaaatcctta cccaactgat gaagacgcgg    1140
   atccgaccga tattgcggac atccaaggcc caacgcagcc aggtacggat gagtctggag    1200
   atttccagag caactccagc gacaacgatt ccacaggcaa cgattccaca ggctctgact    1260
   cttcagattc agattcctcc ggcaacgatt cttcagaggt tatttccggc gatcgttccg    1320
   ctcaga                                                               1326